# EUROPÄISCHE PATENTSCHRIFT

(11) **EP 2 274 271 B1**
(45) Veröffentlichungstag und Bekanntmachung des Hinweises auf die Patenterteilung: **23.01.2013**
(21) Anmeldenummer: 09727743.8
(22) Anmeldetag: 18.03.2009
(51) Int. Cl.: C07C 231/02, C07C 233/05

(54) **VERFAHREN ZUR HERSTELLUNG VON AMIDEN IN GEGENWART VON ÜBERHITZTEM WASSER**
METHOD FOR PRODUCING AMIDES IN THE PRESENCE OF SUPERHEATED WATER
PROCÉDÉ DE PRODUCTION D'AMIDES EN PRÉSENCE D'EAU SURCHAUFFÉE

(30) Priorität: 04.04.2008 DE 102008017219
(43) Veröffentlichungstag der Anmeldung: 19.01.2011
(73) Patentinhaber: Clariant Finance (BVI) Limited, Road Town, Tortola (VG)
(72) Erfinder: KRULL, Matthias, 55296 Harxheim (DE); MORSCHHÄUSER, Roman, 55122 Mainz (DE)
(74) Vertreter: Mikulecky, Klaus
(86) Internationale Anmeldenummer: PCT/EP2009/001988
(87) Internationale Veröffentlichungsnummer: WO 2009/121488

(56) Entgegenhaltungen:
- US-A1- 2008 009 541
- C. FERROUD ET AL: "Microwaves-assisted solvent-free synthesis of N-acetamides by amidation or aminolysis" TETRAHEDRON LETTERS., Bd. 49, 6. März 2008 (2008-03-06), Seiten 3004-3008, XP022602751 NLELSEVIER, AMSTERDAM.
- GORETZKI C ET AL: "GREEN POLYMER CHEMISTRY: MICROWAVE-ASSISTED SINGLE-STEP SYNTHESIS OF VARIOUS (METH)ACRYLAMIDES AND POLY(METH)ACRYLAMIDES DIRECTLY FROM (METH)ACRYLIC ACID AND AMINES" MACROMOLECULAR: RAPID COMMUNICATIONS, WILEY VCH VERLAG, WEINHEIM, DE, Bd. 25, 1. Januar 2004 (2004-01-01), Seiten 513-516, XP009069790 ISSN: 1022-1336 in der Anmeldung erwähnt
- GELENS E ET AL: "An atom efficient and solvent-free synthesis of structurally diverse amides using microwaves" TETRAHEDRON LETTERS, ELSEVIER, AMSTERDAM, Bd. 46, Nr. 21, 23. Mai 2005 (2005-05-23), Seiten 3751-3754, XP025386310 ISSN: 0040-4039 [gefunden am 2005-05-23] in der Anmeldung erwähnt
- VAZQUEZ-TATO M P: "MICROWAVE-MEDIATED SYNTHESIS OF AMIDES" SYNLETT, GEORG THIEME VERLAG, DE, Nr. 7, 1. Januar 1993 (1993-01-01), Seite 506, XP002056049 ISSN: 0936-5214 in der Anmeldung erwähnt
- MASSICOT F ET AL: "Solvent-Free Synthesis of Tartramides Under Microwave Activation" SYNTHESIS, GEORG THIEME VERLAG, STUTTGART, DE, Nr. 16, 1. Januar 2001 (2001-01-01), Seiten 2441-2444, XP002363367 ISSN: 0039-7881

## Beschreibung

Die vorliegende Erfindung betrifft ein Verfahren zur Herstellung von Amiden unter Mikrowellenbestrahlung, wobei das Ammoniumsalz aus mindestens einer Carbonsäure und mindestens einem Amin in Gegenwart von überhitztem Wasser zum Amid kondensiert wird.

Carbonsäureamide finden als chemische Rohstoffe vielseitige Verwendung. Beispielsweise besitzen Carbonsäureamide mit niedrigem Molekulargewicht hervorragende Eigenschaften als Lösemittel, während mindestens einen längeren Alkylrest tragende Carbonsäureamide oberflächenaktiv sind. So werden Carbonsäureamide unter anderem als Lösemittel und als Bestandteil von Wasch- und Reinigungsmitteln sowie in Kosmetika eingesetzt. Weiterhin werden sie erfolgreich als Hilfsmittel in der Metallbearbeitung, bei der Formulierung von Pflanzenschutzmitteln, als Antistatika für Polyolefine sowie bei der Förderung und Verarbeitung von Erdöl verwendet. Darüber hinaus sind Carbonsäureamide auch wichtige Rohstoffe zur Herstellung verschiedenster Pharmazeutika und Agrochemikalien.

Ein neuerer Ansatz zur Synthese von Carbonsäureamiden ist die durch Mikrowellen unterstützte direkte Umsetzung von Carbonsäuren und Aminen zu Amiden. Dabei wird im Gegensatz zu klassischen, thermischen Verfahren keine Aktivierung der Carbonsäure über beispielsweise Säurechloride, Säureanhydride, Ester oder Kupplungsreagentien benötigt, was diese Verfahren ökonomisch wie auch ökologisch sehr interessant macht.

Vázquez-Tato, Synlett 1993, 506, offenbart die Verwendung von Mikrowellen als Heizquelle für die Herstellung von Amiden aus Carbonsäuren und arylaliphatischen Aminen über die Ammoniumsalze.

Gelens et al., Tetrahedron Letters 2005, 46(21), 3751-3754 offenbart eine Vielzahl an Amiden, die unter Zuhilfenahme von Mikrowellenstrahlung synthetisiert wurden.

Goretzki et.al., Macromol. Rapid Commun. 2004, 25, 513-516 offenbart die durch Mikrowellen unterstützte Synthese verschiedener (Meth)acrylamide direkt aus (Meth)acrylsäure und primären Aminen.

Ferroud et al., Tetrahedron Letters 2008, 49,3751-3754 offenbart die Verwendung von Mikrowellen für die Herstellung von Amiden aus Essigsäure und verschiedene Amine.

Die bei den bisher beschriebenen mikrowellenunterstützten Synthesen von Amiden aus Carbonsäure und Amin erreichten Umsätze sind für kommerzielle Anwendungen in der Regel jedoch noch unbefriedigend. So müssen zusätzliche Isolierungs- und Aufarbeitungsschritte durchgeführt werden, um insbesondere unumgesetzte Edukte aus dem Reaktionsgemisch abzutrennen. Da es sich bei Amidierungen um Gleichgewichtsreaktionen handelt, wird zwecks Verschiebung des Gleichgewichts in Richtung des Amids der Gehalt des Reaktionsgemischs an Wasser und insbesondere an Reaktionswasser möglichst niedrig gehalten, was in Batch-Verfahren beispielsweise durch Auskreisen von Wasser mit Schleppmitteln während der Kondensation oder durch Anlegen von Vakuum bewerkstelligt wird. In kontinuierlichen Verfahren, insbesondere bei unter erhöhtem Druck durchgeführten Verfahren ist eine Abtrennung des Reaktionswassers jedoch kaum möglich. Dementsprechend beschreiben Katritzky et al. (Energy & Fuels 4 (1990), 555-561) für aquathermische und An et al. (J. Org. Chem. (1997), 62, 2505-2511) für mikrowellenunterstützte Prozesse in überhitztem Wasser die Hydrolyse von tertiären Amiden zu Carbonsäuren mit teilweise nachfolgender Decarboxylierung. Dabei werden verschiedene Amide wie auch verschiedene Nitrile über die Stufe des Amids zu Carbonsäuren hydrolysiert.

Problematisch bei der Synthese von Amiden aus Carbonsäure und Amin ist oftmals auch die leichte Flüchtigkeit der eingesetzten Edukte, was umfangreiche technische Maßnahmen zu ihrer Handhabung notwendig macht. Weiterhin erfordert die bei der Herstellung der intermediär gebildeten Ammoniumsalze auftretende Neutralisationswärme insbesondere bei leicht flüchtigen Aminen und/oder Carbonsäuren intensives Kühlen und/oder lange Misch- bzw. Reaktionszeiten. Es bestand somit die Aufgabe, ein Verfahren zu entwickeln, mit dem die Umsätze bei mikrowellenunterstützten Amidierungen ausgehend von Carbonsäure und Amin erhöht werden können und bei dem zusätzlich die genannten Nachteile des Standes der Technik vermindert werden.

Überraschenderweise wurde gefunden, dass sich der Umsatz bei Amidierungsreaktionen, bei denen mindestens ein Amin und mindestens eine Carbonsäure zu einem Ammoniumsalz umgesetzt und nachfolgend unter Mikrowellenbestrahlung zum Amid umgesetzt werden, durch die Gegenwart von überhitztem Wasser deutlich erhöhen lässt. Dies war umso überraschender, als derartige, unter Wasserabspaltung verlaufende Kondensationsreaktionen dem Massenwirkungsgesetzt unterliegen und demzufolge die Erhöhung der Konzentration eines der Reaktionsprodukte üblicherweise das Gleichgewicht in Richtung der Edukte verschiebt. Zudem können bei diesem Verfahren wässrige Lösungen insbesondere niedrig siedender Edukte eingesetzt werden, so dass diese nicht unter Druck bzw. in gekühlter Form gehandhabt werden müssen. Weiterhin findet bei der Herstellung des Ammoniumsalzes durch die Gegenwart von Wasser eine verbesserte Wärmeabfuhr statt.

Gegenstand der Erfindung ist ein Verfahren zur Herstellung von Carbonsäureamiden, indem mindestens eine Carbonsäure der Formel I

R³-COOH (I)

worin R³ für Wasserstoff oder einen gegebenenfalls substituierten Kohlenwasserstoffrest mit 1 bis 50 C-Atomen steht,
mit mindestens einem Amin der Formel II

HNR'R² (II)

worin R¹ und R² unabhängig voneinander für Wasserstoff oder einen gegebenenfalls substituierten Kohlenwasserstoffrest mit 1 bis 100 C-Atomen stehen,
zu einem Ammoniumsalz umgesetzt wird, und dieses Ammoniumsalz in Gegenwart von überhitztem Wasser unter Mikrowellenbestrahlung zum Carbonsäureamid umgesetzt wird.

Ein weiterer Gegenstand der Erfindung ist ein Verfahren zur Herstellung von Carbonsäureamiden, indem mindestens eine Carbonsäure der Formel I

R³-COOH (I)

worin R³ für Wasserstoff oder einen gegebenenfalls substituierten Kohlenwasserstoffrest mit 1 bis 50 C-Atomen steht,
mit mindestens einem Amin der Formel II

HNR¹R² (II)

worin R¹ und R² unabhängig voneinander für Wasserstoff oder einen gegebenenfalls substituierten Kohlenwasserstoffrest mit 1 bis 100 C-Atomen stehen,
in Gegenwart von Wasser zu einem Ammoniumsalz umgesetzt wird und das so hergestellte, Wasser enthaltende Ammoniumsalz unter Mikrowellenbestrahlung bei Temperaturen oberhalb 150 °C zum Carbonsäureamid umgesetzt wird.

Ein weiterer Gegenstand der Erfindung ist ein Verfahren zur Erhöhung des Umsatzes von mikrowellenunterstützten Amidierungsreaktionen, bei dem einem Ammoniumsalz aus mindestens einer Carbonsäure der Formel I

R³-COOH (I)

worin R³ für Wasserstoff oder einen gegebenenfalls substituierten Kohlenwasserstoffrest mit 1 bis 50 C-Atomen steht,
und mindestens einem Amin der Formel II

HNR¹R² (II)

worin R¹ und R² unabhängig voneinander für Wasserstoff oder einen gegebenenfalls substituierten Kohlenwasserstoffrest mit 1 bis 100 C-Atomen stehen,
vor der Mikrowellenbestrahlung Wasser zugesetzt wird.

Als Carbonsäuren der Formel I sind allgemein Verbindungen geeignet, die mindestens eine Carboxylgruppe besitzen. So ist das erfindungsgemäße Verfahren ebenso zur Umsetzung von Carbonsäuren mit beispielsweise zwei, drei, vier oder mehr Carboxylgruppen geeignet. Die Carbonsäuren können natürlichen oder synthetischen Ursprungs sein. Besonders bevorzugt sind dabei neben Ameisensäure solche Carbonsäuren, die einen Kohlenwasserstoffrest R³ mit 1 bis 30 C-Atomen und insbesondere mit 2 bis 24 C-Atomen tragen. Der Kohlenwasserstoffrest ist bevorzugt aliphatisch, zykloaliphatisch, aromatisch oder araliphatisch. Der Kohlenwasserstoffrest kann einen oder mehrere wie beispielsweise zwei, drei, vier oder mehr weitere Substituenten wie beispielsweise Hydroxyl-, Hydroxyalkyl-, Alkoxy- wie beispielsweise Methoxy-, Poly(alkoxy)-, Poly(alkoxy)alkyl-, Carboxyl-, Ester-, Amid-, Cyano-, Nitril- Nitro-, Sulfonsäure- und/oder C₅-C₂₀-Arylgruppen wie beispielsweise Phenylgruppen tragen mit der Maßgabe, dass die Substituenten unter den Reaktionsbedingungen stabil sind und keine Nebenreaktionen wie beispielsweise Eliminierungsreaktionen eingehen. Die C₅-C₂₀-Arylgruppen können ihrerseits wiederum Substituenten wie beispielsweise Halogenatome, halogenierte Alkylreste, C₁-C₂₀-Alkyl-, C₁C₂₀-Alkenyl-, C₁-C₅-Alkoxy- wie beispielsweise Methoxy-, Ester-, Amid-, Cyano-, Nitril-, und/oder Nitrogruppen tragen. Der Kohlenwasserstoffrest R³ kann auch Heteroatome wie beispielsweise Sauerstoff, Stickstoff, Phosphor und/oder Schwefel enthalten, bevorzugt jedoch nicht mehr als ein Heteroatom pro 3 C-Atome. Bei der Umsetzung von Polycarbonsäuren mit Ammoniak oder primären Aminen gemäß dem erfindungsgemäßen Verfahren können auch Imide entstehen.

Bevorzugte Carbonsäuren tragen aliphatische Kohlenwasserstoffreste. Besonders bevorzugt sind aliphatische Kohlenwasserstoffreste mit 2 bis 24 und insbesondere mit 3 bis 20 C-Atomen. Diese aliphatischen Kohlenwasserstoffreste können linear, verzweigt oder zyklisch sein. Die Carboxylgruppe kann an einem primären, sekundären oder tertiären C-Atom gebunden sein. Die Kohlenwasserstoffreste können gesättigt oder ungesättigt sein. Ungesättigte Kohlenwasserstoffreste enthalten eine oder mehrere und bevorzugt eine, zwei oder drei C=C-Doppelbindungen. So hat sich das erfindungsgemäße Verfahren besonders zur Herstellung von Amiden ungesättigter und insbesondere mehrfach ungesättigter Fettsäuren bewährt, da die Doppelbindungen der ungesättigten Fettsäuren unter den Reaktionsbedingungen des erfindungsgemäßen Verfahrens nicht angegriffen werden. In einer bevorzugten Ausführungsform ist der aliphatische Kohlenwasserstoffrest ein unsubstituierter Alkyl- oder Alkenylrest. In einer weiteren bevorzugten Ausführungsform trägt der aliphatische Kohlenwasserstoffrest einen oder mehrere wie beispielsweise zwei, drei oder mehr der oben genannten Substituenten.

Bevorzugte zykloaliphatische Kohlenwasserstoffreste sind aliphatische Kohlenwasserstoffreste mit 2 bis 24 und insbesondere mit 3 bis 20 C-Atomen und gegebenenfalls einem oder mehreren Heteroatomen wie beispielsweise Stickstoff, Sauerstoff oder Schwefel, die mindestens einen Ring mit vier, fünf, sechs, sieben, acht oder mehr Ringatomen besitzen. Die Carboxylgruppe ist dabei an einen der Ringe gebunden.

Geeignete aliphatische bzw. zykloaliphatische Carbonsäuren sind beispielsweise Ameisensäure, Essigsäure, Propionsäure, Buttersäure, iso-Buttersäure, Pentansäure, iso-Pentansäure, Pivalinsäure, Hexansäure, Cyclohexansäure, Heptansäure, Octansäure, Nonansäure, Isononansäure, Neononansäure, Decansäure, Isodecansäure, Neodecansäure, Undecansäure, Neoundecansäure, Dodecansäure, Tridecansäure, Tetradecansäure, 12-Methyltridecansäure, Pentadecansäure, 13-Methyltetradecansäure, 12-Methyltetradecansäure, Hexadecansäure, 14-Methylpentadecansäure, Heptadecansäure, 15-Methylhexadecansäure, 14-Methylhexadecansäure, Octadecan-, Isooctadecansäure, Icosansäure, Docosansäure und Tetracosansäure, sowie Myristoleinsäure, Palmitoleinsäure, Hexadecadiensäure, Delta-9-cis-Heptadecensäure, Ölsäure, Petroselinsäure, Vaccensäure, Linolsäure, Linolensäure, Gadoleinsäure, Gondosäure, Icosadiensäure, Arachidonsäure, Cetoleinsäure, Erucasäure, Docosadiensäure und Tetracosensäure sowie Malonsäure, Bernsteinsäure, Butantetracarbonsäure, Dodecenylbernsteinsäure und Octadecenylbernsteinsäure. Weiterhin geeignet sind aus natürlichen Fetten und Ölen wie beispielsweise Baumwollsamen-, Cocos-, Erdnuss-, Färberdistel-, Mais-, Palmkern-, Raps-, Ricinus-, Oliven-, Senfsamen, Soja-, Sonnenblumenöl sowie Talg-, Knochen- und Fischöl gewonnene Fettsäuremischungen. Als Fettsäuren bzw. Fettsäuremischungen für das erfindungsgemäße Verfahren ebenfalls geeignet sind Tallölfettsäure sowie Harz- und Naphthensäuren.

In einer bevorzugten Ausführungsform ist das erfindungsgemäße Verfahren besonders zur Herstellung von Amiden ethylenisch ungesättigter Carbonsäuren, das heißt von Carbonsäuren, die in eine zur Carboxylgruppe konjugierte C=C-Doppelbindung besitzen, geeignet. Beispiele für bevorzugte ethylenisch ungesättigte Carbonsäuren sind Acrylsäure, Methacrylsäure, Crotonsäure, 2,2-Dimethylacrylsäure, Seneciosäure, Maleinsäure, Fumarsäure, Itaconsäure, Zimtsäure und Methoxyzimtsäure

In einer weiteren bevorzugten Ausführungsform ist das erfindungsgemäße Verfahren besonders zur Herstellung von Amiden von Hydroxycarbonsäuren, das heißt von Carbonsäuren, die mindestens eine Hydroxylgruppe am aliphatischen Kohlenwasserstoffrest R³ tragen, geeignet. Die Hydroxylgruppe kann dabei an einem primären, sekundären oder tertiären C-Atom gebunden sein. Besonders vorteilhaft ist das Verfahren für die Amidierung von Hydroxycarbonsäuren, die eine Hydroxylgruppe an einem sekundären C-Atom gebunden enthalten und speziell für die Amidierung von solchen Hydroxycarbonsäuren, bei denen sich die Hydroxylgruppe in α- oder β-Position zur Carboxylgruppe befindet. Carboxyl- und Hydroxylgruppe können an gleiche oder verschiedene C-Atome von R³ gebunden sein. Das erfindungsgemäße Verfahren ist ebenso zur Amidierung von Hydroxypolycarbonsäuren mit beispielsweise zwei, drei, vier oder mehr Carboxylgruppen geeignet. Weiterhin ist das erfindungsgemäße Verfahren zur Amidierung von Polyhydroxycarbonsäuren mit beispielsweise zwei, drei, vier oder mehr Hydroxylgruppen geeignet, wobei die Hydroxycarbonsäuren jedoch nur eine Hydroxylgruppe pro C-Atom des aliphatischen Kohlenwasserstoffrestes R³ tragen dürfen. Besonders bevorzugt sind dabei Hydroxycarbonsäuren, die einen aliphatischen Kohlenwasserstoffrest R³ mit 1 bis 30 C-Atomen und insbesondere mit 2 bis 24 C-Atomeh wie beispielsweise mit 3 bis 20 C-Atomen tragen. Bei der Umsetzung der Hydroxycarbonsäuren nach dem erfindungsgemäßen Verfahren kommt es weder zu Aminolyse oder Elimination der Hydroxylgruppe.

Geeignete aliphatische Hydroxycarbonsäuren sind beispielsweise Hydroxyessigsäure, 2-Hydroxypropionsäure, 3-Hydroxypropionsäure 2-Hydroxybuttersäure, 3-Hydroxybuttersäure, 4-Hydroxybuttersäure, 2-Hydroxy-2-methylpropionsäure, 4-Hydroxypentansäure, 5-Hydroxypentansäure, 2,2-Dimethyl-3-hydroxypropionsäure, 5-Hydroxyhexansäure, 2-Hydroxyoctansäure, 2-Hydroxytetradecansäure, 15-Hydroxypentadecansäure, 16-Hydroxyhexadecansäure, 12-Hydroxystearinsäure sowie α-Hydroxyphenylessigsäure, 4-Hydroxymandelsäure, 2-Hydroxy-2-phenylpropionsäure und 3-Hydroxy-3-phenylpropionsäure. Auch Hydroxypolycarbonsäuren wie beispielsweise Hydroxybernsteinsäure, Zitronensäure und Isozitronensäure, Polyhydroxycarbonsäuren wie beispielsweise Gluconsäure und Polyhydroxypolycarbonsäuren wie beispielsweise Weinsäure lassen sich mittels des erfindungsgemäßen Verfahrens mit erhöhten Umsätzen in die entsprechenden Amide überführen.

Weiterhin bevorzugte Carbonsäuren tragen aromatische Kohlenwasserstoffreste R³ Unter solchen aromatischen Carbonsäuren werden Verbindungen verstanden, die mindestens eine an ein aromatisches System (Arylrest) gebundene Carboxylgruppe tragen. Unter aromatischen Systemen werden zyklische, durchkonjugierte Systeme mit (4n + 2) π-Elektronen verstanden, worin n eine natürliche ganze Zahl und vorzugsweise 1, 2, 3, 4 oder 5 ist. Das aromatische System kann mono- oder polyzyklisch wie beispielsweise di- oder trizyklisch sein. Das aromatische System wird bevorzugt aus Kohlenstoffatomen gebildet. In einer weiteren bevorzugten Ausführungsform enthält es neben Kohlenstoffatomen ein oder mehrere Heteroatome wie beispielsweise Stickstoff, Sauerstoff und/oder Schwefel. Beispiele für solche aromatischen Systeme sind Benzol, Naphthalin, Phenanthren, Furan und Pyridin. Das aromatische System kann neben der Carboxylgruppe ein oder mehrere wie beispielsweise eins, zwei, drei oder mehr gleiche oder verschiedene weitere Substituenten tragen. Geeignete weitere Substituenten sind beispielsweise Alkyl-, Alkenyl- und halogenierte Alkylreste, Hydroxy-, Hydroxyalkyl-, Alkoxy-, Halogen-, Cyano-, Nitril-, Nitro- und/oder Sulfonsäuregruppen. Diese können an beliebiger Position des aromatischen Systems gebunden sein. Der Arylrest trägt jedoch höchstens so viele Substituenten, wie er Valenzen hat.

In einer speziellen Ausführungsform trägt der Arylrest weitere Carboxylgruppen. So ist das erfindungsgemäße Verfahren ebenso zur Umsetzung von aromatischen Carbonsäuren mit beispielsweise zwei oder mehr Carboxylgruppen geeignet. Bei der Umsetzung von Polycarbonsäuren mit Ammoniak oder primären Aminen gemäß dem erfindungsgemäßen Verfahren können, insbesondere wenn sich die Carboxylgruppen in ortho-Stellung an einem aromatischen System befinden, auch Imide entstehen.

Besonders geeignet ist das erfindungsgemäße Verfahren zur Amidierung von Alkylarylcarbonsäuren wie beispielsweise Alkylphenylcarbonsäuren. Dabei handelt es sich um aromatische Carbonsäuren, bei denen der die Carboxylgruppe tragende Arylrest zusätzlich mindestens einen Alkyl- oder Alkylenrest trägt. Besonders vorteilhaft ist das Verfahren bei der Amidierung von Alkylbenzoesäuren, die mindestens einen Alkylrest mit 1 bis 20 C-Atomen und insbesondere 1 bis 12 C-Atomen wie beispielsweise 1 bis 4 C-Atomen tragen.

Weiterhin besonders geeignet ist das erfindungsgemäße Verfahren zur Amidierung von aromatischen Carbonsäuren, deren Arylrest eine oder mehrere wie beispielsweise zwei oder drei Hydroxylgruppen und/oder Hydroxyalkylgruppen trägt. Bei der Amidierung mit mindestens equimolaren Mengen an Amin der Formel (II) findet dabei selektiv eine Amidierung der Carboxylgruppe statt; es werden keine Ester und/oder Polyester gebildet.

Geeignete aromatische Carbonsäuren sind beispielsweise Benzoesäure, Phthalsäure, Isophthalsäure, die verschiedenen Isomeren der Naphthalincarbonsäure, Pyridincarbonsäure und Naphthalindicarbonsäure sowie Trimellitsäure, Trimesinsäure, Pyromellitsäure und Mellitsäure, die verschiedenen Isomere der Methoxybenzoesäure, Hydroxybenzoesäure, Hydroxymethylbenzoesäure, Hydroxymethoxybenzoesäure, Hydroxydimethoxybenzoesäure, Hydroxyisophthalsäure, Hydroxynaphthalincarbonsäure, Hydoxypyridincarbonsäure und Hydroxymethylpyridincarbonsäure, Hydroxychinolincarbonsäure sowie o-Tolylsäure, m-Tolylsäure, p-Tolylsäure, o-Ethylbenzoesäure, m-Ethylbenzoesäure, p-Ethylbenzoesäure, o-Propylbenzoesäure, m-Propylbenzoesäure, p-Propylbenzoesäure und 3,4-Dimethylbenzoesäure.

Weiterhin bevorzugte Carbonsäuren tragen araliphatische Kohlenwasserstoffreste R³. Derartige araliphatische Carbonsäuren tragen mindestens eine über einen Alkylen- oder Alkylenylrest an ein aromatisches System gebundene Carboxylgruppe. Der Alkylen- bzw. Alkenylenrest besitzt dabei bevorzugt 1 bis 10 C-Atome und insbesondere 2 bis 5 C-Atome. Er kann linear oder verzweigt sein, bevorzugt ist er linear. Bevorzugte Alkylenylenreste besitzen eine oder mehrere wie beispielsweise eine, zwei oder drei Doppelbindungen. Unter aromatischem System werden die bereits oben definierten aromatischen Systeme verstanden, an den der mindestens eine Carboxylgruppe tragende Alkylrest gebunden ist. Die aromatischen Systeme können ihrerseits wiederum Substituenten wie beispielsweise Halogenatome, halogenierte Alkylreste, C₁-C₂₀-Alkyl-, C₂-C₂₀-Alkenyl-, C₁-C₅-Alkoxy- wie beispielsweise Methoxy-, Hydroxyl-, Hydroxyalkyl-, Ester-, Amid-, Cyano-, Nitril-, und/oder Nitrogruppen tragen. Beispiele für bevorzugte araliphatische Carbonsäuren sind Phenylessigsäure, (2-Bromphenyl)essigsäure, 3-(Ethoxyphenyl)essigsäure, 4-(Methoxyphenyl)essigsäure, (Dimethoxyphenyl)essigsäure, 2-Phenylpropionsäure, 3-Phenylpropionsäure, 3-(4-Hydroxyphenyl)propionsäure, 4-Hydroxyphenoxyessigsäure, Zimtsäure und deren Mischungen.

Auch Mischungen verschiedener Carbonsäuren sind für den Einsatz im erfindungsgemäßen Verfahren geeignet.

Das erfindungsgemäße Verfahren eignet sich bevorzugt zur Herstellung sekundärer Amide, das heißt zur Umsetzung von Aminen, bei denen R¹ für einen Kohlenwasserstoffrest mit 1 bis 100 Kohlenstoffatomen und R² für Wasserstoff steht.

Das erfindungsgemäße Verfahren eignet sich weiterhin bevorzugt zur Herstellung tertiärer Amine, das heißt zur Umsetzung von Carbonsäuren mit Aminen, bei denen beide Reste R¹ und R² unabhängig voneinander für einen Kohlenwasserstoffrest mit 1 bis 100 Kohlenstoffatomen stehen. Die Reste R¹ und R² können dabei gleich oder verschieden sein. In einer besonders bevorzugten Ausführungsform sind R¹ und R² gleich.

In einer ersten bevorzugten Ausführungsform stehen R¹ und/oder R² unabhängig voneinander für einen aliphatischen Rest. Dieser hat bevorzugt 1 bis 24, besonders bevorzugt 2 bis 18 und speziell 3 bis 6 C-Atome. Der aliphatische Rest kann linear, verzweigt oder zyklisch sein. Er kann weiterhin gesättigt oder ungesättigt sein. Bevorzugt ist der aliphatische Rest gesättigt. Der aliphatische Rest kann Substituenten wie beispielsweise Hydroxy-, C₁-C₅-Alkoxy-, Cyano-, Nitril-, Nitro- und/oder C₅-C₂₀-Arylgruppen wie beispielsweise Phenylreste tragen. Die C₅-C₂₀-Arylreste können ihrerseits gegebenenfalls mit Halogenatomen, halogenierten Alkylresten, C₁-C₂₀-Alkyl-, C₂-C₂₀-Alkenyl-, Hydroxyl-, C₁-C₅-Alkoxy-wie beispielsweise Methoxy-, Amid-, Cyano-, Nitril-, und/oder Nitrogruppen substituiert sein. In einer besonders bevorzugten Ausführungsform stehen R¹ und/oder R² unabhängig voneinander für Wasserstoff, einen C₁-C₆-Alkyl-, C₂-C₆-Alkenyl- oder C₃-C₆-Cycloalkylrest und speziell für einen Alkylrest mit 1, 2, oder 3 C-Atomen. Diese Reste können bis zu drei Substituenten tragen. Besonders bevorzugte aliphatische Reste R¹ und/oder R² sind Wasserstoff, Methyl, Ethyl, Hydroxyethyl, n-Propyl, iso-Propyl, Hydroxypropyl, n-Butyl, iso-Butyl und tert.-Butyl, Hydroxybutyl, n-Hexyl, Cyclohexyl, n-Octyl, n-Decyl, n-Dodecyl, Tridecyl, Isotridecyl, Tetradecyl, Hexadecyl, Octadecyl und Methylphenyl.

In einer weiteren bevorzugten Ausführungsform bilden R¹ und R² zusammen mit dem Stickstoffatom, an das sie gebunden sind, einen Ring. Dieser Ring hat bevorzugt 4 oder mehr wie beispielsweise mit 4, 5, 6 oder mehr Ringglieder. Bevorzugte weitere Ringglieder sind dabei Kohlenstoff-, Stickstoff-, Sauerstoff- und Schwefelatome. Die Ringe können ihrerseits wiederum Substituenten wie beispielsweise Alkylreste tragen. Geeignete Ringstrukturen sind beispielsweise Morpholinyl-, Pyrrolidinyl, Piperidinyl-, Imidazolyl- und Azepanylreste.

In einer weiteren bevorzugten Ausführungsform stehen R¹ und/oder R² unabhängig voneinander für eine gegebenenfalls substituierte C₆-C₁₂-Arylgruppe oder eine gegebenenfalls substituierte heteroaromatische Gruppe mit 5 bis 12 Ringgliedern.

In einer weiteren bevorzugten Ausführungsform stehen R¹ und/oder R² unabhängig voneinander für einen mit Heteroatomen unterbrochenen Alkylrest. Besonders bevorzugte Heteroatome sind Sauerstoff und Stickstoff.

So stehen R¹ und/oder R² unabhängig voneinander bevorzugt für Reste der Formel III

-(R⁴-O)ₙ-R⁵ (III)

worin
- R⁴: für eine Alkylengruppe mit 2 bis 6 C-Atomen und bevorzugt mit 2 bis 4 C-Atomen wie beispielsweise Ethylen, Propylen, Butylen oder Mischungen daraus,
- R⁵: für Wasserstoff, einen Kohlenwasserstoffrest mit 1 bis 24 C-Atomen oder eine Gruppe der Formel -NR¹⁰R¹¹,
- n: für eine Zahl zwischen 2 und 50, bevorzugt zwischen 3 und 25 und insbesondere zwischen 4 und 10 und
- R¹⁰, R¹¹: unabhängig voneinander für Wasserstoff, einen aliphatischen Rest mit 1 bis 24 C-Atomen und bevorzugt 2 bis 18 C-Atomen, eine Arylgruppe- oder Heteroarylgruppe mit 5 bis 12 Ringgliedern, eine Poly(oxyalkylen)gruppe mit 1 bis 50 Poly(oxyalkylen)einheiten, wobei sich die Polyoxyalkyleneinheiten von Alyklenoxideinheiten mit 2 bis 6 C-Atomen ableiten, oder R¹⁰ und R¹¹gemeinsam mit dem Stickstoffatom, an das sie gebunden sind, einen Ring mit 4, 5, 6 oder mehr Ringgliedern bilden, stehen.

Weiterhin bevorzugt stehen R¹ und/oder R² unabhängig voneinander für Reste der Formel IV

-[R⁶-N(R⁷)]ₘ-(R⁷) (IV)

worin
- R⁶: für eine Alkylengruppe mit 2 bis 6 C-Atomen und bevorzugt mit 2 bis 4 C-Atomen wie beispielsweise Ethylen, Propylen oder Mischungen daraus steht,
- jedes R⁷: unabhängig voneinander für Wasserstoff, einen Alkyl- oder Hydroxyalkylrest mit bis zu 24 C-Atomen wie beispielsweise 2 bis 20 C-Atomen, einen Polyoxyalkylenrest -(R⁴-O)ₚ-R⁵, oder einen Polyiminoalkylenrest -[R⁶-N(R⁷)]_{q}-(R⁷) stehen, wobei R⁴, R⁵, R⁶ und R⁷ die oben gegebenen Bedeutungen haben und q und p unabhängig voneinander für 1 bis 50 stehen und
- m: für eine Zahl von 1 bis 20 und bevorzugt 2 bis 10 wie beispielsweise drei, vier, fünf oder sechs steht. Die Reste der Formel IV enthalten vorzugsweise 1 bis 50, insbesondere 2 bis 20 Stickstoffatome.

Je nach stöchiometrischem Verhältnis zwischen aromatischer Carbonsäure (I) und Polyamin (IV) werden eine oder mehrere Aminogruppen, die jeweils mindestens ein Wasserstoffatom tragen, in das Carbonsäureamid überführt. Bei der Umsetzung von Polycarbonsäuren mit Polyaminen der Formel IV können insbesondere die primären Aminogruppen auch in Imide überführt werden.

Zur erfindungsgemäßen Herstellung von primären Amiden werden anstelle von Ammoniak bevorzugt stickstoffhaltige Verbindungen, die beim Erhitzen Ammoniakgas abspalten, eingesetzt. Beispiele für derartige stickstoffhaltige Verbindungen sind Harnstoff und Formamid.

Beispiele für geeignete Amine sind Ammoniak, Methylamin, Ethylamin, Ethanolamin, Propylamin, Propanolamin, Butylamin, Hexylamin, Cyclohexylamin, Octylamin, Decylamin, Dodecylamin, Tetradecylamin, Hexadecylamin, Octadecylamin, Dimethylamin, Diethylamin, Diethanolamin, Ethylmethylamin, Di-n-propylamin, Di-iso-propylamin, Dicyclohexylamin, Didecylamin, Didodecylamin, Ditetradecylamin, Dihexadecylamin, Dioctadedcylamin, Benzylamin, Phenylethylamin, Ethylendiamin, Diethylentriamin, Triethylentetramin, Tetraethylenpentamin, N,N-Dimethylethylendiamin, N,N-Diethylaminopropylamin, N,N-Dimethylaminopropylamin, N,N-(2'-hydroxyethyl)-1,3-propandiamin und 1-(3-Aminopropyl)pyrrolidin sowie deren Mischungen. Besonders bevorzugt sind davon Dimethylamin, Diethylamin, Diethanolamin, Di-n-propylamin, Di-iso-propylamin, Ethylmethylamin und N,N-Dimethylaminopropylamin.

Besonders geeignet ist das erfindungsgemäße Verfahren zur Herstellung von Amiden aus gesättigten C₁-C₅-Carbonsäuren und primären Alkyl- und/oder Arylaminen, aus gesättigten C₁-C₅-Carbonsäuren und sekundären Alkyl- und/oder Arylaminen, aus gesättigten C₁-C₅-Carbonsäuren und Hydroxylgruppen tragenden Aminen, aus gesättigten C₁-C₅-Carbonsäuren und Polyetheraminen, aus gesättigten C₁-C₅-Carbonsäuren und Polyaminen, aus aliphatischen Hydroxycarbonsäuren und primären Alkyl- und/oder Arylaminen, aus aliphatischen Hydroxycarbonsäuren und sekundären Alkyl- und/oder Arylaminen, aus aliphatischen Hydroxycarbonsäuren und Polyaminen, aus C₆-C₅₀-Alkyl- und/oder Alkenylcarbonsäuren und Polyetheraminen, aus C₆-C₅₀-Alkyl- und/oder Alkenylcarbonsäuren und Polyaminen, aus C₆-C₅₀-Alkyl- und/oder Alkenylcarbonsäuren und primären Alkyl- und/oder Arylaminen, aus C₆-C₅₀-Alkyl- und/oder Alkenylcarbonsäuren und sekundären Alkyl- und/oder Arylaminen, aus C₆-C₅₀-Alkyl- und/oder Alkenylcarbonsäuren und Hydroxylgruppen tragenden Aminen, aus C₃-C₅-Alkenylcarbonsäuren und primären Alkyl- und/oder Arylaminen, aus C₃-C₅-Alkenylcarbonsäuren und sekundären Alkyl- und/oder Arylaminen, aus C₃-C₅-Alkenylcarbonsäuren und Hydroxylgruppen tragenden Aminen, aus C₃-C₅-Alkenylcarbonsäuren und Polyetheraminen, aus C₃-C₅-Alkenylcarbonsäuren und Polyaminen, aus gegebenenfalls Hydroxylgruppen tragenden Arylcarbonsäuren und primären Alkyl- und/oder Arylaminen, aus gegebenenfalls Hydroxylgruppen tragenden Arylcarbonsäuren und sekundären Alkyl- und/oder Arylaminen, aus gegebenenfalls Hydroxylgruppen tragenden Arylcarbonsäuren und Hydroxylgruppen tragenden Aminen, aus gegebenenfalls Hydroxylgruppen tragenden Arylcarbonsäuren und Polyetheraminen sowie aus gegebenenfalls Hydroxylgruppen tragenden Arylcarbonsäuren und Polyaminen.

Das Verfahren ist insbesondere geeignet zur Herstellung von N,N-Dimethylformamid, N-Octylformamid, N-Methylacetamid, N,N-Dimethylacetamid, N-Ethylacetamid, N,N-Diethylacetamid, N,N-Dipropylacetamid, N,N-Dimethylpropionsäureamid, N,N-Dimethylbuttersäureamid, N,N-Dimethyl(phenyl)essigsäureamid, N,N-Dimethylmilchsäureamid, N,N-Dimethylacrylamid, N,N-Dimethylacrylamid, N,N-Diethymethacrylamid, N,N-Diethyacrylamid, N-2-Ethylhexylacrylamid, N-2-Ethylhexylmethacrylamid, N-Methylcocosfettsäureamid, N,N-Dimethylcocosfettsäureamid, N-Methylglycolsäureamid, N-Ethylmandelsäureamid, N,N-Dimethylglykolsäureamid, N,N-Dimethylmilchsäureamid, N,N-Dimethylricinolsäureamid, Octansäurediethanolamid, Laurinsäuremonoethanolamid, Laurinsäurediethanolamid, Tallölfettsäurediethanolamid, Tallölfettsäuremonoethanolamid, N,N-Dimethylbenzamid, N,N-Diethylbenzamid, Nicotinsäureamid, N,N-Dimethylnicotinsäureamid, N,N-Diethyl-Tolylsäureamid und N,N'-Di(essigsäure)-ethylendiamid.

Im erfindungsgemäßen Verfahren können Carbonsäure und Amin in der Regel in beliebigen Verhältnissen miteinander zur Reaktion gebracht werden. Bevorzugt erfolgt die Umsetzung mit molaren Verhältnissen zwischen Carbonsäure und Amin von 10:1 bis 1:100, bevorzugt von 2:1 bis 1:10, speziell von 1,2:1 bis 1:3, jeweils bezogen auf die Equivalente an Carboxyl- und Aminogruppen. In einer speziellen Ausführungsform werden Carbonsäure und Amin equimolar eingesetzt. In vielen Fällen hat es sich als vorteilhaft erwiesen, mit einem Überschuss an Amin, das heißt molaren Verhältnissen von Amin zu Carbonsäure von mindestens 1,01 : 1,00 und insbesondere zwischen 1,02 : 1,00 und 5,0 : 1,0 wie beispielsweise zwischen 2,5 : 1,0 und 1,1 : 1,0 zu arbeiten. Besonders vorteilhaft ist dieses Verfahren, wenn das eingesetzte Amin leicht flüchtig oder wasserlöslich ist. Leicht flüchtig heißt hier, dass das Amin einen Siedepunkt bei Normaldruck von vorzugsweise unterhalb 250 °C wie beispielsweise unterhalb 150 °C besitzt und sich somit, gegebenenfalls gemeinsam mit dem Wasser, vom Amid abtrennen lässt. Dies kann beispielsweise mittels Phasentrennung, Extraktion oder Destillation erfolgen.

Für den Fall, dass R¹ und/oder R² für einen mit einer oder mehreren Hydroxylgruppen substituierten Kohlenwasserstoffrest stehen, erfolgt die Umsetzung zwischen Carbonsäure (I) und Amin (II) mit molaren Verhältnissen von 1:1 bis 1:100, bevorzugt von 1:1,001 bis 1:10 und speziell von 1:1,01 bis 1:5 wie beispielsweise von 1:1,1 bis 1:2, jeweils bezogen auf die Molequivalente an Carboxylgruppen und Aminogruppen im Reaktionsgemisch.

Für den Fall, dass die Carbonsäure (I) eine oder mehrere Hydroxylgruppen trägt, erfolgt die Umsetzung zwischen Carbonsäure (I) und Amin (II) mit molaren Verhältnissen von 1:100 bis 1:1, bevorzugt von 1:10 bis 1:1,001 und speziell von 1:5 bis 1:1,01 wie beispielsweise von 1:2 bis 1:1,1, jeweils bezogen auf die Molequivalente an Carboxylgruppen und Aminogruppen im Reaktionsgemisch.

Für den Fall, dass R¹ und/oder R² für einen mit einer oder mehreren Hydroxylgruppen substituierten Kohlenwasserstoffrest stehen und dass die Carbonsäure eine oder mehrere Hydroxylgruppen trägt, erfolgt die Umsetzung zwischen Carbonsäure (I) und Amin (II) equimolar bezogen auf die Molequivalente an Carboxylgruppen und Aminogruppen im Reaktionsgemisch.

Die Umsetzung von Amin und Carbonsäure zum Ammoniumsalz kann kontinuierlich, diskontinuierlich oder auch in semi-Batch-Prozessen durchgeführt werden. So kann die Herstellung des Ammoniumsalzes direkt in dem für die Mikrowellenbestrahlung vorgesehenen Reaktionsgefäß (Bestrahlungsgefäß) erfolgen. Sie kann auch in einem vorgelagerten (semi)-Batch Prozess durchgeführt werden wie beispielsweise in einem separaten Rührbehälter. Das Ammoniumsalz wird bevorzugt in-situ erzeugt und nicht isoliert. So hat es sich insbesondere für Prozesse im industriellen Maßstab bewährt, die Umsetzung von Amin und Carbonsäure in Gegenwart von Wasser zum Ammoniumsalz in einer Mischstrecke vorzunehmen, aus der das Wasser enthaltende Ammoniumsalz, gegebenenfalls nach Zwischenkühlung, in das Bestrahlungsgefäß gefördert wird. Dabei kann das Wasser als separater Stoffstrom oder bevorzugt als Löse- oder Dispergiermittel für Amin und/oder Carbonsäure der Mischstrecke zugeführt werden. Weiterhin bevorzugt werden die Edukte dem erfindungsgemäßen Verfahren in flüssiger Form zugeführt. Dazu können höher schmelzende und/oder höher viskose Edukte beispielsweise in geschmolzenem Zustand und/oder mit Wasser und/oder weiterem Lösemittel versetzt beispielsweise als Lösung, Dispersion oder Emulsion eingesetzt werden. Ein Katalysator kann, sofern eingesetzt, einem der Edukte oder auch der Eduktmischung vor dem Eintritt in das Bestrahlungsgefäß zugesetzt werden. Auch feste, pulverförmige und heterogene Systeme können nach dem erfindungsgemäßen Verfahren umgesetzt werden, wobei lediglich entsprechende technische Vorrichtungen zum Fördern des Reaktionsgutes erforderlich sind.

Unter der Gegenwart von Wasser wird erfindungsgemäß verstanden, dass dem aus Carbonsäure und Amin gebildeten Ammoniumsalz vor der Bestrahlung mit Mikrowellen Wasser zugesetzt wird und somit die mikrowellenunterstützte Umsetzung zum Amid in Gegenwart von Wasser stattfindet. Folglich enthält das Reaktionsprodukt eine über die Menge des bei der Amidbildung freiwerdenden Reaktionswasser hinausgehende Menge Wasser. Bevorzugt werden dem Reaktionsgemisch 0,1 bis 5.000 Gew.-%, besonders bevorzugt 1 bis 1.000 Gew.-% und insbesondere 5 bis 100 Gew.-% wie beispielsweise 10 bis 50 Gew.-% Wasser bezogen auf die Gesamtmasse an Carbonsäure und Amin zugesetzt. In einer besonders bevorzugten Ausführungsform wird mindestens eines der Edukte Carbonsäure und/oder Amin als wässrige Lösung zur Bildung des Ammoniumsalzes eingesetzt. So hat es sich beispielsweise bewährt, insbesondere unterhalb Raumtemperatur siedende Amine wie beispielsweise Ammoniak, Methylamin, Dimethylamin oder Ethylamin als beispielsweise 40-70 %ige wässrige Lösungen zur Herstellung des Ammoniumsalzes einzusetzen. Die wässrige Einstellung des Ammoniumsalzes wird nachfolgend, gegebenenfalls nach weiterer Zugabe von Wasser, Mikrowellenstrahlung ausgesetzt.

Überhitztes Wasser wird erfindungsgemäß dadurch erhalten, dass die Mikrowellenbestrahlung unter Bedingungen durchgeführt wird, bei denen Wasser unter Druck auf Temperaturen oberhalb 150 °C erhitzt wird. Bevorzugt wird die Amidierung in Gegenwart von Wasser bei Temperaturen zwischen 180 und 500 °C und insbesondere zwischen 200 und 400 °C wie beispielsweise zwischen 220 und 350 °C durchgeführt. Diese Temperaturen beziehen sich auf die während der Mikrowellenbestrahlung maximal erreichten Temperaturen. Der Druck wird dabei bevorzugt so hoch eingestellt, dass das Reaktionsgemisch im flüssigen Zustand ist und nicht siedet. Bevorzugt wird bei Drücken oberhalb 1 bar, bevorzugt bei Drücken zwischen 3 und 300 bar, besonderes bevorzugt zwischen 5 und 200 und insbesondere zwischen 10 und 100 bar wie beispielsweise zwischen 15 und 50 bar gearbeitet.

Zur Beschleunigung bzw. zur Vervollständigung der Reaktion hat es sich in vielen Fällen bewährt, in Gegenwart von dehydratisierenden Katalysatoren zu arbeiten. Unter dehydratisierenden Katalysatoren werden Hilfsstoffe verstanden, die die Kondensation von Amin und Carbonsäure beschleunigen. Vorzugsweise arbeitet man dabei in Gegenwart eines sauren anorganischen, metallorganischen oder organischen Katalysators oder Gemischen aus mehreren dieser Katalysatoren. In einer besonders bevorzugten Ausführungsform wird ohne Katalysator gearbeitet.

In einer bevorzugten Ausführungsform wird in Gegenwart zusätzlicher organischer Lösemittel gearbeitet um beispielsweise die Viskosität des Reaktionsmediums abzusenken und/oder das Reaktionsgemisch, sofern es heterogen ist, zu fluidisieren. Dafür können prinzipiell alle Lösemittel eingesetzt werden, die unter den angewendeten Reaktionsbedingungen inert sind und nicht mit den Edukten bzw. den gebildeten Produkten reagieren. Sofern in Gegenwart von zusätzlichen Lösemitteln gearbeitet wird, liegt deren Anteil an der Reaktionsmischung bevorzugt zwischen 1 und 90 Gew.-%, speziell zwischen 5 und 75 Gew.-% und insbesondere zwischen 10 und 60 Gew.-% wie beispielsweise zwischen 20 und 50 Gew.-%. Besonders bevorzugt wird die Reaktion in Abwesenheit zusätzlicher Lösemittel durchgeführt.

Nach der Mikrowellenbestrahlung kann das Reaktionsgemisch in vielen Fällen direkt einer weiteren Verwendung zugeführt werden. Um wasserfreie Produkte zu erhalten, kann das Wasser durch übliche Trennverfahren wie beispielsweise Phasentrennung, Destillation, Gefriertrocknung oder Absorption vom Rohprodukt abgetrennt werden. Dabei können auch im Überschuss eingesetzte Edukte sowie gegebenenfalls nicht umgesetzte Restmengen der Edukte mit abgetrennt werden. Für spezielle Anforderungen können die Rohprodukte nach üblichen Reinigungsverfahren wie beispielsweise Destillation, Umkristallisation, Filtration bzw. chromatographische Verfahren weiter aufgereinigt werden.

Die Mikrowellenbestrahlung wird üblicherweise in Geräten durchgeführt, die einen Reaktionsraum (Bestrahlungsgefäß) aus einem für Mikrowellen weitestgehend transparenten Material besitzen, in den in einem Mikrowellengenerator erzeugte Mikrowellenstrahlung eingekoppelt wird. Mikrowellengeneratoren, wie beispielsweise das Magnetron, das Klystron und das Gyrotron sind dem Fachmann bekannt.

Die zur Durchführung des erfindungsgemäßen Verfahrens eingesetzten Bestrahlungsgefäße sind bevorzugt aus weitgehend mikrowellentransparentem, hoch schmelzendem Material gefertigt oder enthalten zumindest Teile wie beispielsweise Fenster aus diesen Materialien. Besonders bevorzugt werden nichtmetallische Bestrahlungsgefäße eingesetzt. Unter weitgehend mikrowellentransparent werden hier Werkstoffe verstanden, die möglichst wenig Mikrowellenenergie absorbieren und in Wärme umwandeln. Als Maß für die Fähigkeit eines Stoffes, Mikrowellenenergie zu absorbieren und in Wärme zu überführen wird oftmals der dielektrische Verlustfaktor tan δ = ε''/ε' herangezogen. Der dielektrische Verlustfaktor tan δ ist definiert als das Verhältnis aus dielektrischem Verlust ε'' und Dielektrizitätskonstante ε'. Beispiele für tan δ-Werte verschiedener Materialien sind beispielsweise in D. Bogdal, Microwave-assisted Organic Synthesis, Elsevier 2005 wiedergegeben. Für erfindungsgemäß geeignete Bestrahlungsgefäße werden Materialen mit bei 2,45 GHz und 25 °C gemessenen tan δ-Werten von unter 0,01, insbesondere unter 0,005 und speziell unter 0,001 bevorzugt. Als bevorzugte mikrowellentransparente und temperaturstabile Materialien kommen in erster Linie Werkstoffe auf mineralischer Basis wie beispielsweise Quarz, Aluminiumoxid, Zirkonoxid und ähnliches in Betracht. Auch temperaturstabile Kunststoffe wie insbesondere Fluorpolymere wie beispielsweise Teflon, und technische Kunststoffe wie Polypropylen, oder Polyaryletherketone wie beispielsweise glasfaserverstärktes Polyetheretherketon (PEEK) sind als Gefäßmaterialien geeignet. Um den Temperaturbedingungen während der Reaktion zu widerstehen haben sich insbesondere mit diesen Kunststoffen beschichtete Mineralien wie Quarz oder Aluminiumoxid als Reaktormaterialien bewährt.

Als Mikrowellen werden elektromagnetische Strahlen mit einer Wellenlänge zwischen etwa 1 cm und 1m und Frequenzen zwischen etwa 300 MHz und 30 GHz bezeichnet. Dieser Frequenzbereich ist prinzipiell für das erfindungsgemäße Verfahren geeignet. Bevorzugt wird für das erfindungsgemäße Verfahren Mikrowellenstrahlung mit für industrielle, wissenschaftliche und medizinische Anwendungen freigegebenen Frequenzen verwendet wie beispielsweise mit Frequenzen von 915 MHz, 2,45 GHz, 5,8 GHz oder 27,12 GHz. Die Mikrowellenbestrahlung des Ammoniumsalzes kann sowohl in Mikrowellenapplikatoren, die im Mono- bzw. Quasi-Monomode arbeiten wie auch in solchen, die im Multimode arbeiten, erfolgen. Entsprechende Geräte sind dem Fachmann bekannt.

Die für die Durchführung des erfindungsgemäßen Verfahrens in das Bestrahlungsgefäß einzustrahlende Mikrowellenleistung ist insbesondere abhängig von der angestrebten Reaktionstemperatur, der Geometrie des Reaktionsraums und damit des Reaktionsvolumens. Sie liegt üblicherweise zwischen 100 W und mehreren 100 kW und insbesondere zwischen 200 W und 100 kW wie beispielsweise zwischen 500 W und 70 kW. Sie kann an einer oder mehreren Stellen des Bestrahlungsgefäßes appliziert werden. Sie kann über einen oder mehrere Mikrowellengeneratoren erzeugt werden.

Die Dauer der Mikrowellenbestrahlung hängt von verschiedenen Faktoren wie dem Reaktionsvolumen, der Geometrie des Bestrahlungsgefäßes, der gewünschten Verweilzeit des Reaktionsgemisches bei Reaktionstemperatur sowie dem gewünschten Umsetzungsgrad ab. Üblicherweise wird die Mikrowellenbestrahlung über einen Zeitraum von weniger als 30 Minuten, bevorzugt zwischen 0,01 Sekunde und 15 Minuten, besonders bevorzugt zwischen 0,1 Sekunde und 10 Minuten und insbesondere zwischen einer Sekunde und 5 Minuten wie beispielsweise zwischen 5 Sekunden und 2 Minuten vorgenommen. Die Intensität (Leistung) der Mikrowellenstrahlung wird dabei so eingestellt, dass das Reaktionsgut in möglichst kurzer Zeit die angestrebte Reaktionstemperatur erreicht. In einer weiteren bevorzugten Ausführungsform des erfindungsgemäßen Verfahrens hat es sich bewährt, das Ammoniumsalz schon vor Beginn der Mikrowellenbestrahlung zu erwärmen, wozu unter anderem die bei der Bildung des Ammoniumsalzes frei werdende Reaktionswärme genutzt werden kann. Besonders bewährt hat sich dabei ein Aufheizen des Ammoniumsalzes auf Temperaturen zwischen etwa 40 und etwa 120 °C, bevorzugt jedoch auf Temperaturen unterhalb des Siedepunkts des Systems. Zum Aufrechterhalten der angestrebten Reaktionstemperatur kann das Reaktionsgut mit reduzierter und/oder gepulster Leistung weiter bestrahlt oder anderweitig auf Temperatur gehalten werden. In einer bevorzugten Ausführungsform wird das Umsetzungsprodukt direkt nach Beendigung der Mikrowellenbestrahlung möglichst schnell auf Temperaturen unterhalb 120 °C, bevorzugt unterhalb 100 °C und speziell unterhalb 50 °C abgekühlt.

Die Mikrowellenbestrahlung kann diskontinuierlich im Batch-Verfahren oder, bevorzugt, kontinuierlich zum Beispiel in einem Strömungsrohr durchgeführt werden. Sie kann weiterhin in semi-Batch Prozessen wie beispielsweise kontinuierlich betriebenen Rührreaktoren oder Kaskadenreaktoren durchgeführt werden. In einer bevorzugten Ausführungsform wird die Reaktion in einem geschlossenen, druckfesten und chemisch inerten Gefäß durchgeführt, wobei das Wasser sowie gegebenenfalls die Edukte zu einem Druckaufbau führen. Nach Beendigung der Reaktion kann der Überdruck durch Entspannen zur Verflüchtigung und Abtrennung von Wasser sowie gegebenenfalls überschüssigen Edukten und/oder Abkühlung des Reaktionsprodukts verwendet werden. In einer weiteren Ausführungsform wird das Wasser nach dem Abkühlen und/oder Entspannen durch übliche Verfahren wie beispielsweise Phasentrennung, Destillation und/oder Absorption abgetrennt. In einer besonders bevorzugten Ausführungsform wird das Reaktionsgemisch nach Beenden der Mikrowellenbestrahlung bzw. nach Verlassen des Bestrahlungsgefäßes möglichst schnell von überschüssigem Amin und Wasser befreit, um eine Hydrolyse des Amids zu vermeiden. Dies kann beispielsweise durch übliche Trennverfahren wie Phasentrennung, Destillation oder Absorption erfolgen. Oftmals hat es sich hierbei auch als erfolgreich erwiesen, das Amin zu neutralisieren oder mit überschüssiger Säure zu versetzen. Dabei werden bevorzugt pH-Werte unterhalb 7 wie beispielsweise zwischen 1 und 6,5 und insbesondere zwischen 3 und 6 eingestellt.

In einer bevorzugten Ausführungsform wird das erfindungsgemäße Verfahren in einem diskontinuierlichen Mikrowellenreaktor durchgeführt, in dem eine bestimmte Menge des wässrigen Ammoniumsalzes in ein Bestrahlungsgefäß gefüllt, mit Mikrowellen bestrahlt und anschließend aufgearbeitet wird. Dabei wird die Mikrowellenbestrahlung bevorzugt in einem druckfesten, gerührten Gefäß vorgenommen. Die Einkopplung der Mikrowellen in das Reaktionsgefäß kann, sofern das Reaktionsgefäß aus einem für Mikrowellen transparenten Material gefertigt ist oder für Mikrowellen transparente Fenster besitzt, über die Gefäßwandung erfolgen. Die Mikrowellen können aber auch über Antennen, Sonden bzw. Hohlleitersysteme in das Reaktionsgefäß eingekoppelt werden. Für die Bestrahlung größerer Reaktionsvolumina wird die Mikrowelle hier bevorzugt im Multimode betrieben. Die diskontinuierliche Ausführungsform des erfindungsgemäßen Verfahrens erlaubt durch Variation der Mikrowellenleistung schnelle wie auch langsame Heizraten und insbesondere das Halten der Temperatur über längere Zeiträume wie beispielsweise mehrere Stunden. In einer bevorzugten Ausführungsform wird das wässrige Reaktionsgemisch vor Beginn der Mikrowellenbestrahlung im Bestrahlungsgefäß vorgelegt. Bevorzugt hat es dabei Temperaturen unterhalb 100 °C wie beispielsweise zwischen 10 und 50 °C. In einer weiteren bevorzugten Ausführungsform werden die Reaktanden und Wasser oder Teile davon dem Bestrahlungsgefäß erst während der Bestrahlung mit Mikrowellen zugeführt. In einer weiteren bevorzugten Ausführungsform wird der diskontinuierliche Mikrowellenreaktor unter kontinuierlichem Zuführen von Edukten und gleichzeitigem Ausschleusen von Reaktionsgut in Form eines Semi-Batch- bzw. Kaskadenreaktors betrieben.

In einer besonders bevorzugten Ausführungsform wird das erfindungsgemäße Verfahren in einem kontinuierlichen Mikrowellenreaktor durchgeführt. Die Reaktionsmischung wird dazu kontinuierlich durch ein druckfestes, gegenüber den Reaktanden inertes, für Mikrowellen weitestgehend transparentes und in einen Mikrowellenapplikator eingebautes, als Bestrahlungsgefäß dienendes Reaktionsrohr geführt. Dieses Reaktionsrohr hat bevorzugt einen Durchmesser von einem Millimeter bis ca. 50 cm, speziell zwischen 2 mm und 35 cm wie beispielsweise zwischen 5 mm und 15 cm. Unter Reaktionsrohren werden hier Bestrahlungsgefäß verstanden, deren Verhältnis von Länge zu Durchmesser größer als 5, bevorzugt zwischen 10 und 100.000, besonders bevorzugt zwischen 20 und 10.000 wie beispielsweise zwischen 30 und 1.000 ist. In einer speziellen Ausführungsform ist das Reaktionsrohr in Form eines Doppelmantelrohres ausgestaltet, durch dessen Innen- und Außenraum die Reaktionsmischung nacheinander im Gegenstrom geführt werden kann, um beispielsweise die Temperaturführung und Energieeffizienz des Verfahrens zu erhöhen. Als Länge des Reaktionsrohres ist dabei die vom Reaktionsgemisch insgesamt durchströmte Strecke zu verstehen. Das Reaktionsrohr ist auf seiner Länge von mindestens einem, bevorzugt aber von mehreren wie beispielsweise zwei, drei, vier, fünf, sechs, sieben acht oder mehr Mikrowellenstrahlern umgeben. Die Mikrowelleneinstrahlung erfolgt bevorzugt über den Rohrmantel. In einer weiteren bevorzugten Ausführungsform erfolgt die Mikrowelleneinstrahlung mittels mindestens einer Antenne über die Rohrenden.

Das Reaktionsrohr ist üblicherweise am Einlass mit einer Dosierpumpe sowie einem Manometer und am Auslass mit einem Druckhalteventil und einem Wärmetauscher versehen. Bevorzugt wird das Wasser enthaltende Ammoniumsalz dem Reaktionsrohr in flüssiger Form mit Temperaturen unterhalb 150 °C wie beispielsweise zwischen 10 °C und 90 °C zugeführt. In einer weiteren bevorzugten Ausführungsform werden Amin, Carbonsäure, von denen mindestens eine Komponente Wasser enthält, erst kurz vor dem Eintritt in das Reaktionsrohr vermischt. Weiterhin bevorzugt werden die Edukte dem erfindungsgemäßen Verfahren in flüssiger Form mit Temperaturen unterhalb 100 °C wie beispielsweise zwischen 10 °C und 50 °C zugeführt. Dazu können höher schmelzende Edukte beispielsweise in geschmolzenem Zustand oder mit Lösemittel versetzt eingesetzt werden.

Durch Variation von Rohrquerschnitt, Länge der Bestrahlungszone (hierunter wird der Anteil des Reaktionsrohres verstanden, in dem das Reaktionsgut Mikrowellenstrahlung ausgesetzt ist), Fließgeschwindigkeit, Geometrie der Mikrowellenstrahler, der eingestrahlten Mikrowellenleistung sowie der dabei erreichten Temperatur werden die Reaktionsbedingungen so eingestellt, dass die maximale Reaktionstemperatur schnellstmöglich erreicht wird. In einer bevorzugten Ausführungsform wird die Verweilzeit bei Maximaltemperatur so kurz gewählt, dass so wenig Neben- oder Folgereaktionen wie möglich auftreten. Bevorzugt wird der kontinuierliche Mikrowellenreaktor im Monomode oder Quasi-Monomode betrieben. Die Verweilzeit im Reaktionsrohr liegt dabei im Allgemeinen unter 20 Minuten, bevorzugt zwischen 0,01 Sekunden und 10 Minuten, bevorzugt zwischen 0,1 Sekunden und 5 Minuten wie beispielsweise zwischen einer Sekunde und 3 Minuten. Das Reaktionsgut kann zur Vervollständigung der Reaktion, gegebenenfalls nach Zwischenkühlung, mehrfach das Reaktionsrohr durchlaufen.

In einer besonders bevorzugten Ausführungsform erfolgt die Bestrahlung des wässrigen Ammoniumsalzes mit Mikrowellen in einem Reaktionsrohr, dessen Längsachse sich in der Ausbreitungsrichtung der Mikrowellen in einem Monomode-Mikrowellenapplikator befindet. Insbesondere erfolgt die Bestrahlung des Salzes mit Mikrowellen dabei in einem weitgehend mikrowellentransparenten Reaktionsrohr, das sich innerhalb eines mit einem Mikrowellengenerator verbundenen, als Mikrowellenapplikator fungierenden Hohlleiters befindet. Bevorzugt fluchtet das Reaktionsrohr axial mit einer zentralen Symmetrieachse dieses Hohlleiters. Der Hohlleiter ist bevorzugt als Hohlraumresonator ausgeformt. Weiterhin bevorzugt werden die im Hohlleiter nicht absorbierten Mikrowellen an seinem Ende reflektiert. Durch Ausformung des Mikrowellenapplikators als Resonator vom Reflexionstyp werden eine lokale Erhöhung der elektrischen Feldstärke bei gleicher vom Generator zugeführter Leistung und eine erhöhte Energieausnutzung erzielt.

Der Hohlraumresonator wird bevorzugt im E₀₁ₙ-Mode betrieben, wobei n für eine ganze Zahl steht und die Anzahl der Feldmaxima der Mikrowelle entlang der zentralen Symmetrieachse des Resonators angibt. Bei diesem Betrieb ist das elektrische Feld in Richtung der zentralen Symmetrieachse des Hohlraumresonators gerichtet. Es hat im Bereich der zentralen Symmetrieachse ein Maximum und nimmt zur Mantelfläche hin auf den Wert null ab. Diese Feldkonfiguration liegt rotationssymmetrisch um die zentrale Symmetrieachse vor. Je nach der gewünschten Strömungsgeschwindigkeit des Reaktionsguts durch das Reaktionsrohr, der benötigten Temperatur und der benötigten Verweilzeit im Resonator wird die Länge des Resonators relativ zu der Wellenlänge der eingesetzten Mikrowellenstrahlung ausgewählt. Bevorzugt ist n eine ganze Zahl von 1 bis 200, besonders bevorzugt von 2 bis 100, insbesondere von 4 bis 50 speziell von 3 bis 20 wie beispielsweise 3, 4, 5, 6, 7 oder 8.

Die Einstrahlung der Mikrowellenenergie in den als Mikrowellenapplikator fungierenden Hohlleiter kann über geeignet dimensionierte Löcher oder Schlitze erfolgen. In einer speziellen Ausführungsform des erfindungsgemäßen Verfahrens erfolgt die Bestrahlung des Ammoniumsalzes mit Mikrowellen in einem Reaktionsrohr, das sich in einem Hohlleiter mit koaxialem Übergang der Mikrowellen befindet. Für dieses Verfahren besonders bevorzugte Mikrowelleneinrichtungen sind aus einem Hohlraumresonator, einer Koppeleinrichtung zum Einkoppeln eines Mikrowellenfeldes in den Hohlraumresonator und mit je einer Öffnung an zwei gegenüber liegenden Stirnwänden zum Hindurchführen des Reaktionsrohres durch den Resonator aufgebaut. Die Einkopplung der Mikrowellen in den Hohlraumresonator erfolgt bevorzugt über einen Koppelstift, der in den Hohlraumresonator hineinragt. Bevorzugt ist der Koppelstift als ein als Kopplungsantenne fungierendes, bevorzugt metallisches Innenleiterrohr ausgeformt. In einer besonders bevorzugten Ausführungsform ragt dieser Koppelstift durch eine der stirnseitigen Öffnungen in den Hohlraumresonator hinein. Besonders bevorzugt schließt sich das Reaktionsrohr an das Innenleiterrohr des koaxialen Übergangs an und speziell wird es durch dessen Hohlraum hindurch in den Hohlraumresonator geführt. Bevorzugt fluchtet das Reaktionsrohr axial mit einer zentralen Symmetrieachse des Hohlraumresonators, wozu der Hohlraumresonator bevorzugt je eine zentrische Öffnung an zwei gegenüber liegenden Stirnwänden zum Hindurchführen des Reaktionsrohres aufweist.

Die Einspeisung der Mikrowellen in den Koppelstift bzw. in das als Kopplungsantenne fungierende Innenleiterrohr kann beispielsweise mittels einer koaxialen Anschlussleitung erfolgen. In einer bevorzugten Ausführungsform wird das Mikrowellenfeld über einen Hohlleiter dem Resonator zugeführt, wobei das aus dem Hohlraumresonator herausragende Ende des Koppelstifts in eine Öffnung, die sich in der Wand des Hohlleiters befindet, in den Hohlleiter hineingeführt ist und von dem Hohlleiter Mikrowellenenergie entnimmt und in den Resonator koppelt.

In einer speziellen Ausführungsform erfolgt die Bestrahlung des Salzes mit Mikrowellen in einem mikrowellentransparenten Reaktionsrohr, das sich axialsymmetrisch in einem E₀₁ₙ-Rundhohlleiter mit koaxialem Übergang der Mikrowellen befindet. Dabei wird das Reaktionsrohr durch den Hohlraum eines als Kopplungsantenne fungierenden Innenleiterrohres in den Hohlraumresonator geführt. In einer weiteren bevorzugten Ausführungsform erfolgt die Bestrahlung des Salzes mit Mikrowellen in einem mikrowellentransparenten Reaktionsrohr, das durch einen E₀₁ₙ-Hohlraumresonator mit axialer Einspeisung der Mikrowellen geführt wird, wobei die Länge des Hohlraumresonators so bemessen ist, dass sich n = 2 oder mehr Feldmaxima der Mikrowelle ausbilden. In einer weiteren bevorzugten Ausführungsform erfolgt die Bestrahlung des Salzes mit Mikrowellen in einem mikrowellentransparenten Reaktionsrohr, das sich axialsymmetrisch in einem kreiszylindrischen E₀₁ₙ-Hohlraumresonator mit koaxialem Übergang der Mikrowellen befindet, wobei die Länge des Hohlraumresonators so bemessen ist, dass sich n = 2 oder mehr Feldmaxima der Mikrowelle ausbilden.

Für das erfindungsgemäße Verfahren besonders geeignete E₀₁-Hohlraumresonatoren haben bevorzugt einen Durchmesser, der mindestens der halben Wellenlänge der verwendeten Mikrowellenstrahlung entspricht. Bevorzugt beträgt der Durchmesser des Hohlraumresonators das 1,0- bis 10-fache, besonders bevorzugt das 1,1- bis 5-fache und insbesondere das 2,1- bis 2,6-fache der halben Wellenlänge der verwendeten Mikrowellenstrahlung. Bevorzugt hat der E₀₁-Hohlraumresonator einen runden Querschnitt, was auch als E₀₁-Rundhohlleiter bezeichnet wird. Besonders bevorzugt hat er eine zylindrische Form und speziell eine kreiszylindrische Form.

Die Vorteile des erfindungsgemäßen Verfahrens liegen zum einen in einem erhöhten Umsatz der eingesetzten Edukte gegenüber einer Umsetzung unter vergleichbaren Bedingungen ohne Zusatz von Wasser. So wird der Umsatz durch Zusatz von Wasser üblicherweise um mehr als 1 mol-%, vielfach um mehr als 5 mol-%, teilweise um mehr als 10 mol-% wie beispielsweise um mehr als 20 mol-% erhöht. Das heißt, es verbleiben weniger Edukte im Reaktionsgemisch, die abgetrennt und aufgearbeitet bzw. entsorgt werden müssen. In vielen Fällen ist es sogar gelungen, durch das erfindungsgemäße Arbeiten in Gegenwart von Wasser Amide in direkt vermarktbaren Qualitäten zu erhalten. Weiterhin ist die Handhabung speziell niedrig siedender Carbonsäuren und/oder Amine in Form wässriger Lösungen wesentlich einfacher und sicherer als das Arbeiten mit entsprechenden Gasen. Bei der Bildung des Ammoniumsalzes aus Carbonsäure und Amin frei werdende Neutralisationswärme wird zudem vom Wasser zumindest teilweise aufgenommen und lässt sich leichter abführen als aus organischen Lösemitteln. Darüber hinaus wirkt die Anwesenheit des Wassers als Lösemittel einer Kristallisation der Ammoniumsalze entgegen, so dass auf aufwendige Beheizung von Reaktionsgemisch enthaltenden Leitungen und Gefäßen vor und nach der Mikrowellenbestrahlung verzichtet werden kann.

### Beispiele

Die Mikrowellenbestrahlung erfolgt in einem Single-Mode Mikrowellenreaktor vom Typ "Initiator^{®}" der Firma Biotage bei einer Frequenz von 2,45 GHz. Die Temperaturmessung erfolgte über einen IR-Sensor. Als Reaktionsgefäße dienten geschlossene, druckfeste Glasküvetten (Druckviole) mit einem Volumen von 5 ml, in denen mit Magnetrührung homogenisiert wurde. Die Temperaturmessung erfolgt über einen IR-Sensor.

Die Mikrowellenleistung wurde über die Versuchsdauer jeweils in der Art eingestellt, dass die gewünschte Temperatur des Reaktionsguts so schnell wie möglich erreicht und anschließend über den in den Versuchsbeschreibungen angegebenen Zeitraum konstant gehalten wurde. Nach Beendigung der Mikrowellenbestrahlung wurde die Glasküvette mit Druckluft abgekühlt.

Die Analytik der Reaktionsprodukte erfolgte mittels ¹H-NMR-Spektroskopie bei 500 MHz in CDCl₃.

### Beispiel 1: Herstellung von N,N-Dimethyllactamid

In einem 500 ml Dreihalskolben mit Gaseinleitungsrohr, Rührer, Innenthermometer und Druckausgleich wurden 100 g Lactol 90^{®} (1 mol Milchsäure als 90 %ig wässrige Einstellung) vorgelegt. Unter Eiskühlung wurden langsam 45,1 g gasförmiges Dimethylamin (1 mol) in den Kolben eingeleitet, worauf hin sich in einer stark exothermen Reaktion das Milchsäure-N,N-dimethylammonium-Salz bildete.

Von dieser Stammlösung wurden Aliquots entnommen und durch Zugabe von Wasser auf die in Tabelle 1 angegebenen Wassergehalte eingestellt. Jeweils 2 ml dieser Lösungen wurden im Mikrowellenreaktor auf eine Temperatur von 225 °C erhitzt, wobei sich ein Druck von etwa 20 bar einstellte. Nach Erreichen des thermischen Gleichgewichts (nach ca. 1 Minute) wurde zwei Minuten lang unter weiterer Mikrowellenbestrahlung bei dieser Temperatur und diesem Druck gehalten. Mittels ¹H-NMR-Signalintegration wurden die relativen Anteile von Edukten und Produkt im Reaktionsgemisch ermittelt. Die Umsetzungsraten sind in der letzten Spalte von Tabelle 1 wiedergegeben.

**Tabelle 1:**

| Reaktion | Milchsäure-N,N.-dimethylammonium-Salz | Wasser [Gew.-%] | Molverhältnis Säure:Amin | Umsatz zu N,N-Dimethyllactamid |
|---|---|---|---|---|
| (1) | 93 Gew% | 7 | 1:1 | 35 mol-% |
| (2) | 64 Gew% | 36 | 1:1 | 48 mol-% |
| (3) | 56 Gew% | 44 | 1:1 | 66 mol-% |
| (4) | 47 Gew% | 53 | 1:1 | 90 mol-% |
| (5) | 31 Gew% | 69 | 1:1 | 94 mol-% |

### Beispiel 2: Herstellung von 4-Methoxyphenylessigsäure-N,N-dimethylamid

In einem 500 ml Dreihalskolben mit Gaseinleitungsrohr, Rührer, Innenthermometer und Druckausgleich wurden 166,2 g 4 Methoxyphenylessigsäure (1 mol) vorgelegt und unter Kühlung langsam mit 112,5 g Dimethylamin (als 40 %ig wässrige Lösung) neutralisiert. In einer stark exothermen Reaktion bildete sich das N,N-Dimethylammonium-Salz der 4-Methoxyphenylessigsäure. Der Feststoffgehalt der wässrigen Lösung dieses Salzes betrug 76 %. Von einem Aliquot dieser Lösung wurde durch weitere Wasserzugabe eine Verdünnung des Salzes auf 50 % vorgenommen.

Neben den wässrigen Lösungen wurde zum Vergleich das wasserfreie Ammoniumsalz hergestellt und unter gleichen Bedingungen Mikrowellenstrahlung ausgesetzt. Hierzu wurden in einer Druckviole 1,66 g 4-Methoxyphenylessigsäure unter Trockeneiskühlung vorgelegt und anschließend zügig mittels einer durch Trockeneis vorgekühlten Glaspipette mit 0,45 g kondensiertem Dimethylamin versetzt. Die Viole wurde sofort verschlossen und anschließend langsam aufgetaut, wobei sich in exothermer Reaktion das 4-Methoxyphenylessigsäure-N,N-dimethylammonium-Salz bildete. Zur Homogenisierung der Salzbildung wurde anschließend stark geschüttelt und mit einem Magnetrührfisch gerührt.

Jeweils 2 ml des Ammoniumsalzes bzw. seiner wässrigen Lösungen wurden im Mikrowellenreaktor auf eine Temperatur von 235 °C erhitzt, wobei sich ein Druck von etwa 20 bar einstellte. Nach Erreichen des thermischen Gleichgewichts (nach ca. 1 Minute) wurden die Proben zehn Minuten lang unter weiterer Mikrowellenbestrahlung bei dieser Temperatur und diesem Druck gehalten. Mittels ¹H-NMR-Signalintegration wurden die relativen Anteile von Edukten und Produkt im Reaktionsgemisch ermittelt. Die erzielten Umsetzungsraten sind in der letzten Spalte von Tabelle 2 wiedergegeben.

**Tabelle 2:**

| Reaktion | 4-Methoxyphenylessigsäure-N,N-dimethylammonium-Salz | Wasser [Gew.-%] | Molverhältnis Säure : Amin | Umsatz zu N,N-Dimethyl-(4-Methoxyphenyl)-acetamid |
|---|---|---|---|---|
| (6) | 100 Gew.-% | 0 | 1:1 | 8 % mol-% |
| (7) | 76 Gew.-% | 24 | 1:1 | 25 % mol% |
| (8) | 50 Gew.-% | 50 | 1:1 | 41 % mol-% |

### Beispiel 3: Herstellung von Decansäure-N,N.dimethylamid

In einem 500 ml Dreihalskolben mit Gaseinleitungsrohr, Rührer, Innenthermometer und Druckausgleich wurden 172 g Decansäure (1 mol) vorgelegt und vorsichtig mit 112,5 g Dimethylamin (als 40 %ig wässrige Lösung) neutralisiert. In einer exothermen Reaktion bildete sich das Decansäure-N,N-dimethylammonium-Salz. Der Feststoffgehalt der pastösen, wässrigen Formulierung des Salzes betrug 76 Gew.-%. Von einem Aliquot dieser Lösung wurde durch weitere Wasserzugabe eine Verdünnung des Salzes auf 55 Gew.-% vorgenommen.

Neben den wässrigen Lösungen wurde zum Vergleich das wasserfreie Ammoniumsalz hergestellt und unter gleichen Bedingungen Mikrowellenstrahlung ausgesetzt. In einer Druckviole wurden 1,72 g Decansäure (0,01 mol) unter Trockeneiskühlung vorgelegt und anschließend zügig mittels einer durch Trockeneis vorgekühlten Glaspipette mit 0,45 g kondensiertem Dimethylamin (0,01 mol) versetzt. Die Viole wurde sofort verschlossen und anschließend unter Wasserkühlung vorsichtig aufgetaut, wobei sich das Decansäure-N,N-dimethylammonium-Salz bildete. Zur Vervollständigung der Salzbildung wurde stark geschüttelt und mit einem Magnetrührfisch gerührt.

Jeweils 2 ml des Ammoniumsalzes bzw. seiner wässrigen Lösungen wurden im Mikrowellenreaktor auf eine Temperatur von 240 °C erhitzt, wobei sich ein Druck von etwa 20 bar einstellte. Nach Erreichen des thermischen Gleichgewichts (nach ca. 1 Minute) wurden die Proben zehn Minuten lang unter weiterer Mikrowellenbestrahlung bei dieser Temperatur und diesem Druck gehalten. Mittels ¹H-NMR-Signalintegration wurden die relativen Anteile von Edukten und Produkt im Reaktionsgemisch ermittelt. Die erzielten Umsetzungsraten sind in der letzten Spalte von Tabelle 3 wiedergegeben.

**Tabelle 3:**

| Reaktion | Dekansäure-N,N.-dimethylammonium-Salz | Wasser [Gew.-%] | Molverhältnis Säure: Amin | Umsatz zu N,N.-Dimethyldekansäureamid |
|---|---|---|---|---|
| (9) | 100 Gew.-% | 0 | 1:1 | 15 mol-% |
| (10) | 65 Gew.-% | 35 | 1:1 | 26 mol-% |
| (11) | 49 Gew.-% | 51 | 1:1 | 35 mol-% |

### Beispiel 4: Herstellung von m-Tolylsäure-diethylamid

In einem 500 ml Dreihalskolben mit Gaseinleitungsrohr, Rührer, Innenthermometer und Druckausgleich wurden 136,2 g m-Tolylsäure (1 mol) vorgelegt und vorsichtig mit 109,71 g Diethylamin (1,5 mol) neutralisiert. In einer stark exothermen Reaktion bildete sich das m-Tolylsäure-N,N-diethylammonium-Salz. Von dieser Stammlösung wurden Aliquots entnommen und durch Zugabe von Wasser auf die in Tabelle 4 angegebenen Wassergehalte eingestellt.

Jeweils 2 ml des Ammoniumsalzes bzw. seiner wässrigen Lösungen wurden im Mikrowellenreaktor auf eine Temperatur von 250 °C erhitzt, wobei sich ein Druck von etwa 20 bar einstellte. Nach Erreichen des thermischen Gleichgewichts (nach ca. 1 Minute) wurden die Proben 20 Minuten lang unter weiterer Mikrowellenbestrahlung bei dieser Temperatur und diesem Druck gehalten. Mittels ¹H-NMR-Signalintegration wurden die relativen Anteile von Edukten und Produkt im Reaktionsgemisch ermittelt. Die erzielten Umsetzungsraten sind in der letzten Spalte von Tabelle 4 wiedergegeben.

**Tabelle 4:**

| Reaktion | m-Tolylsäure-N,N.-dimethylammonium-Salz | Wasser [Gew%] | Molverhältnis Säure: Amin | Umsatz zu Dekansäure-N,N.-dimethylamid |
|---|---|---|---|---|
| (12) | 100 Gew.-% | 0 | 1:1,5 | 5 mol-% |
| (13) | 75 Gew.-% | 25 | 1:1.5 | 15 mol-% |
| (14) | 65 Gew.-% | 35 | 1:1.5 | 19 mol-% |
| (15) | 51 Gew.-% | 49 | 1:1.5 | 22 mol-% |

## Patentansprüche

1. Verfahren zur Herstellung von Carbonsäureamiden, indem mindestens eine Carbonsäure der Formel I
R³-COOH (1)
worin R³ für Wasserstoff oder einen gegebenenfalls substituierten Kohlenwasserstoffrest mit 1 bis 50 C-Atomen steht,
mit mindestens einem Amin der Formel II
HNR¹R² (II)
worin R¹ und R² unabhängig voneinander für Wasserstoff oder einen gegebenenfalls substituierten Kohlenwasserstoffrest mit 1 bis 100 C-Atomen stehen, oder R¹ und R² zusammen mit dem Stickstoffatom, an das sie gebunden sind, einen Ring bilden, zu einem Ammoniumsalz umgesetzt wird, und dieses Ammoniumsalz in Gegenwart von überhitztem Wasser unter Mikrowellenbestrahlung zum Carbonsäureamid umgesetzt wird, wobei dem aus Carbonsäure und Amin gebildeten Ammoniumsalz vor der Bestrahlung mit Mikrowellen Wasser zugesetzt wird, wobei die Mikrowellenbestrahlung bei Temperaturen oberhalb 150 °C durchgeführt wird.

2. Verfahren zur Herstellung von Carbonsäureamiden, indem mindestens eine Carbonsäure der Formel I
R³-COOH (I)
worin R³ für Wasserstoff oder einen gegebenenfalls substituierten Kohlenwasserstoffrest mit 1 bis 50 C-Atomen steht,
mit mindestens einem Amin der Formel II
HNR¹R² (II)
worin R¹ und R² unabhängig voneinander für Wasserstoff oder einen gegebenenfalls substituierten Kohlenwasserstoffrest mit 1 bis 100 C-Atomen stehen, oder R¹ und R² zusammen mit dem Stickstoffatom, an das sie gebunden sind, einen Ring bilden, in Gegenwart von Wasser zu einem Ammoniumsalz umgesetzt wird und das so hergestellte Wasser enthaltende Ammoniumsalz unter Mikrowellenbestrahlung bei Temperaturen oberhalb 150 °C zum Carbonsäureamid umgesetzt wird.

3. Verfahren zur Erhöhung des Umsatzes von mikrowellenunterstützten Amidierungsreaktionen, bei dem einem Ammoniumsalz aus mindestens einer Carbonsäure der Formel I
R³-COOH (I)
worin R³ für Wasserstoff oder einen gegebenenfalls substituierten Kohlenwasserstoffrest mit 1 bis 50 C-Atomen steht,
und mindestens einem Amin der Formel II
HNR¹R² (II)
worin R¹ und R² unabhängig voneinander für Wasserstoff oder einen gegebenenfalls substituierten Kohlenwasserstoffrest mit 1 bis 100 C-Atomen stehen,
vor der Mikrowellenbestrahlung Wasser zugesetzt wird, wobei die Mikrowellenbestrahlung bei Temperaturen oberhalb 150 °C durchgeführt wird.

4. Verfahren nach einem oder mehreren der Ansprüche 1 bis 3, bei dem die Mikrowellenbestrahlung bei Drücken oberhalb des Atmosphärendrucks erfolgt.

5. Verfahren nach einem oder mehreren der Ansprüche 1 bis 4, bei dem R³ ein Kohlenwasserstoffrest mit 1 bis 50 C-Atomen ist, der einen oder mehrere Substituenten ausgewählt aus C₁-C₅-Alkoxy-, Poly(C₁-C₅-Alkoxy)-, Poly(C₁-C₅-Alkoxy)alkyl-, Carboxyl-, Hydroxyl-, Ester-, Amid-, Cyano-, Nitril-, Nitro-, Sulfonsäure- und Arylgruppen mit 5 bis 20 Kohlenstoffatomen trägt, wobei die C₅-C₂₀-Arylgruppen Substituenten ausgewählt aus Halogenatomen, halogenierten Alkylresten, C₁-C₂₀-Alkyl-, C₂-C₂₀-Alkenyl-, C₁-C₅-Alkoxy -, Ester-, Amid-, Hydroxy-, Hydroxyalkyl-, Cyano-, Nitril-, und Nitrogruppen tragen können.

6. Verfahren nach einem oder mehreren der Ansprüche 1 bis 5, bei dem R³ ein aliphatischer, cycloaliphatischer, aromatischer oder araliphatischer Kohlenwasserstoffrest ist.

7. Verfahren nach einem oder mehreren der Ansprüche 1 bis 6, bei dem R³ eine oder mehrere Doppelbindungen umfasst.

8. Verfahren nach einem oder mehreren der Ansprüche 1 bis 7, bei dem R¹ und R² unabhängig voneinander für einen Kohlenwasserstoffrest mit 1 bis 100 Kohlenstoffatomen stehen.

9. Verfahren nach einem oder mehreren der Ansprüche 1 bis 8, bei dem R¹ für einen Kohlenwasserstoffrest mit 1 bis 100 Kohlenstoffatomen und R² für Wasserstoff steht.

10. Verfahren nach einem oder mehreren der Ansprüche 1 bis 9, bei dem R¹ oder R² oder beide Reste unabhängig voneinander für einen aliphatischen Rest mit 1 bis 24 C-Atomen stehen.

11. Verfahren nach einem oder mehreren der Ansprüche 1 bis 10, bei dem R¹ oder R² oder beide Reste Substituenten ausgewählt aus Hydroxy-, C₁-C₅-Alkoxy-, Cyano-, Nitril-, Nitro- und C₅-C₂₀-Arylgruppen tragen.

12. Verfahren nach einem oder mehreren der Ansprüche 1 bis 11, bei dem R¹ oder R² oder beide Reste C₅-C₂₀-Arylgruppen tragen, und diese einen oder mehrere Substituenten ausgewählt aus Halogenatomen, halogenierten Alkylresten, C₁-C₂₀-Alkyl-, C₂-C₂₀-Alkenyl-, C₁-C₅-Alkoxy-, Ester-, Amid-, Cyano-, Nitril-, und Nitrogruppen tragen.

13. Verfahren nach einem oder mehreren der Ansprüche 1 bis 12, bei dem R¹ und R² zusammen mit dem Stickstoffatom, an das sie gebunden sind, einen Ring bilden.

14. Verfahren nach einem oder mehreren der Ansprüche 1 bis 13, bei dem R¹ und R² unabhängig voneinander für Reste der Formel III
-(R⁴-O)ₙ-R⁵ (III)
stehen, worin
R⁴ für eine Alkylengruppe mit 2 bis 6 C-Atomen,
R⁵ für Wasserstoff oder einen Kohlenwasserstoffrest mit 1 bis 24 C-Atomen, und
n für eine Zahl zwischen 2 und 50 stehen.

15. Verfahren nach einem oder mehreren der Ansprüche 1 bis 14, bei dem R¹ und R² unabhängig voneinander für Reste der Formel IV
-[R⁶-N(R⁷)]ₘ-(R⁷) (IV)
stehen, worin
R⁶ für eine Alkylengruppe mit 2 bis 6 C-Atomen oder Mischungen daraus,
jedes R⁷ unabhängig voneinander für Wasserstoff, einen Alkyl- oder Hydroxyalkylrest mit bis zu 24 C-Atomen, einen Polyoxyalkylenrest -(R⁴-O)ₚ-R⁵, oder einen Polyiminoalkylenrest -[R⁶-N(R⁷)]_{q}-(R⁷), wobei R⁴, R⁵, R⁶ und R⁷ die oben angegebenen Bedeutungen haben und q und p unabhängig voneinander für 1 bis 50 stehen, und
m für eine Zahl von 1 bis 20 und bevorzugt 2 bis 10 wie beispielsweise drei, vier, fünf oder sechs stehen.

16. Verfahren nach einem oder mehreren der Ansprüche 1 bis 15, bei dem die Bestrahlung des Salzes mit Mikrowellen in einem diskontinuierlichen Batch-Verfahren erfolgt.

17. Verfahren nach einem oder mehreren der Ansprüche 1 bis 16, bei dem die Bestrahlung des Salzes mit Mikrowellen in einem kontinuierlichen Verfahren erfolgt.

18. Verfahren nach Anspruch 17, bei dem die Bestrahlung des Salzes mit Mikrowellen in einem weitgehend mikrowellentransparenten Reaktionsrohr erfolgt.

19. Verfahren nach Anspruch 17 und 18, bei dem die Bestrahlung des Salzes mit Mikrowellen in einem Reaktionsrohr erfolgt, dessen Längsachse sich in der Ausbreitungsrichtung der Mikrowellen eines Monomode-Mikrowellenapplikators befindet.

20. Verfahren nach einem oder mehreren der Ansprüche 1 bis 19, wobei die Mikrowellenbestrahlung in Gegenwart von 0,5 bis 200 Gew.-% Wasser bezogen auf die Gesamtmasse an Carbonsäure und Amin durchgeführt wird.

21. Verfahren nach einem oder mehreren der Ansprüche 1 bis 20, bei dem die Mikrowellenbestrahlung bei Temperaturen oberhalb 180 °C durchgeführt wird.

## Claims

1. A process for preparing carboxamides by reacting at least one carboxylic acid of the formula I
R³-COOH (I)
in which R³ is hydrogen or an optionally substituted hydrocarbon radical having 1 to 50 carbon atoms
with at least one amine of the formula II
HNR¹R² (II)
in which R¹ and R² are each independently hydrogen or an optionally substituted hydrocarbon radical having 1 to 100 carbon atoms, or R¹ and R² together with the nitrogen atom to which they are bonded form a ring,
to give an ammonium salt, and this ammonium salt is converted to the carboxamide in the presence of superheated water under microwave irradiation, wherein water is added before the irradiation with microwaves to the ammonium salt formed from carboxylic acid and amine, the microwave irradiation being performed at temperatures above 150°C.

2. A process for preparing carboxamides by reacting at least one carboxylic acid of the formula I
R³-COOH (I)
in which R³ is hydrogen or an optionally substituted hydrocarbon radical having 1 to 50 carbon atoms
with at least one amine of the formula II
HNR¹R² (II)
in which R¹ and R² are each independently hydrogen or an optionally substituted hydrocarbon radical having 1 to 100 carbon atoms, or R¹ and R² together with the nitrogen atom to which they are bonded form a ring,
in the presence of water to give an ammonium salt, and the water-containing ammonium salt thus prepared is converted to the carboxamide at temperatures above 150°C under microwave irradiation.

3. A process for increasing the conversion of microwave-supported amidation reactions, in which water is added before microwave irradiation to an ammonium salt of at least one carboxylic acid of the formula I
R³-COOH (I)
in which R³ is hydrogen or an optionally substituted hydrocarbon radical having 1 to 50 carbon atoms
and at least one amine of the formula II
HNR¹R² (II)
in which R¹ and R² are each independently hydrogen or an optionally substituted hydrocarbon radical having 1 to 100 carbon atoms, wherein the microwave irradiation is performed at temperatures above 150°C.

4. The process as claimed in one or more of claims 1 to 3, in which the microwave irradiation is effected at pressures above atmospheric pressure.

5. The process as claimed in one or more of claims 1 to 4, in which R³ is a hydrocarbon radical which has 1 to 50 carbon atoms and bears one or more substituents selected from C₁-C₅-alkoxy, poly(C₁-C₅-alkoxy), poly(C₁-C₅-alkoxy)-alkyl, carboxyl, hydroxyl, ester, amide, cyano, nitrile, nitro, sulfo and aryl groups having 5 to 20 carbon atoms, where the C₅-C₂₀-aryl groups may bear substituents selected from halogen atoms, halogenated alkyl radicals, C₁-C₂₀-alkyl, C₂-C₂₀-alkenyl, C₁-C₅-alkoxy, ester, amide, hydroxyl, hydroxyalkyl, cyano, nitrile and nitro groups.

6. The process as claimed in one or more of claims 1 to 5, in which R³ is an aliphatic, cycloaliphatic, aromatic or araliphatic hydrocarbon radical.

7. The process as claimed in one or more of claims 1 to 6, in which R³ comprises one or more double bonds.

8. The process as claimed in one or more of claims 1 to 7, in which R¹ and R² are each independently a hydrocarbon radical having 1 to 100 carbon atoms.

9. The process as claimed in one or more of claims 1 to 8, in which R¹ is a hydrocarbon radical having 1 to 100 carbon atoms and R² is hydrogen.

10. The process as claimed in one or more of claims 1 to 9, in which R¹ or R² is, or both radicals are each independently, an aliphatic radical having 1 to 24 carbon atoms.

11. The process as claimed in one or more of claims 1 to 10, in which R¹ and R² is, or both radicals are, substituents selected from hydroxyl, C₁-C₅-alkoxy, cyano, nitrile, nitro and C₅-C₂₀-aryl groups.

12. The process as claimed in one or more of claims 1 to 11, in which R¹ or R² bears, or both radicals bear, C₅-C₂₀-aryl groups which bear one or more substituents selected from halogen atoms, halogenated alkyl radicals, C₁-C₂₀-alkyl, C₂-C₂₀-alkenyl, C₁-C₅-alkoxy, ester, amide, cyano, nitrile and nitro groups.

13. The process as claimed in one or more of claims 1 to 12, in which R¹ and R² together with the nitrogen atom to which they are bonded form a ring.

14. The process as claimed in one or more of claims 1 to 13, in which R¹ and R² are each independently radicals of the formula III
-(R⁴-O)ₙ-R⁵ (III)
in which
R⁴ is an alkylene group having 2 to 6 carbon atoms,
R⁵ is hydrogen or a hydrocarbon radical having 1 to 24 carbon atoms, and
n is from 2 to 50.

15. The process as claimed in one or more of claims 1 to 14, in which R¹ and R² are each independently radicals of the formula IV
-[R⁶-N(R⁷)]ₘ-(R⁷) (IV)
in which
R⁶ is an alkylene group having 2 to 6 carbon atoms or mixtures thereof,
each R⁷ is independently hydrogen, an alkyl or hydroxyalkyl radical having up to 24 carbon atoms, a polyoxyalkylene radical -(R⁴-O)ₚ-R⁵ or a polyimino-alkylene radical -[R⁶-N(R⁷)]_{q}-(R⁷) where R⁴, R⁵, R⁶ and R⁷ are each as defined above and q and p are each independently 1 to 50, and
m is from 1 to 20 and preferably 2 to 10, for example three, four, five or six.

16. The process as claimed in one or more of claims 1 to 15, in which the salt is irradiated with microwaves in a batchwise process.

17. The process as claimed in one or more of claims 1 to 16, in which the salt is irradiated with microwaves in a continuous process.

18. The process as claimed in claim 17, in which the salt is irradiated with microwaves in a substantially microwave-transparent reaction tube.

19. The process as claimed in claims 17 and 18, in which the salt is irradiated with microwaves in a reaction tube whose longitudinal axis is in the direction of propagation of the microwaves of a monomode microwave applicator.

20. The process as claimed in one or more of claims 1 to 19, wherein the microwave irradiation is performed in the presence of 0.5 to 200% by weight of water based on the total mass of carboxylic acid and amine.

21. The process as claimed in one or more of claims 1 to 20, in which the microwave irradiation is performed at temperatures above 180°C.

## Revendications

1. Procédé pour la préparation d'amides d'acide carboxylique, en transformant au moins un acide carboxylique de formule I
R³-COOH (I)
dans laquelle R³ représente hydrogène ou un radical hydrocarboné le cas échéant substitué comprenant 1 à 50 atomes de carbone, avec au moins une amine de formule II
HNR¹R² (II)
dans laquelle R¹ et R² représentent, indépendamment l'un de l'autre, hydrogène ou un radical hydrocarboné le cas échéant substitué comprenant 1 à 100 atomes de carbone, ou R¹ et R² forment ensemble avec l'atome d'azote auquel ils sont liés, un cycle, en un sel d'ammonium et on transforme ce sel d'ammonium en présence d'eau surchauffée sous irradiation par des micro-ondes en amide d'acide carboxylique, le sel d'ammonium formé à partir de l'acide carboxylique et de l'amine étant additionné d'eau avant l'irradiation par des micro-ondes, l'irradiation par des micro-ondes étant réalisée à des températures supérieures à 150°C.

2. Procédé pour la préparation d'amides d'acide carboxylique, en transformant au moins un acide carboxylique de formule I
R³-COOH (I)
dans laquelle R³ représente hydrogène ou un radical hydrocarboné le cas échéant substitué comprenant 1 à 50 atomes de carbone, avec au moins une amine de formule II
HNR¹R² (II)
dans laquelle R¹ et R² représentent, indépendamment l'un de l'autre, hydrogène ou un radical hydrocarboné le cas échéant substitué comprenant 1 à 100 atomes de carbone, ou R¹ et R² forment ensemble avec l'atome d'azote auquel ils sont liés, un cycle, en présence d'eau en un sel d'ammonium et on transforme le sel d'ammonium ainsi préparé, contenant de l'eau, sous irradiation par des micro-ondes à des températures supérieures à 150°C en amide d'acide carboxylique.

3. Procédé pour augmenter la conversion de réactions d'amidation soutenues par des micro-ondes, dans lequel un sel d'ammonium formé à partir d'au moins un acide carboxylique de formule I
R³-COOH (I)
dans laquelle R³ représente hydrogène ou un radical hydrocarboné le cas échéant substitué comprenant 1 à 50 atomes de carbone, et d'au moins une amine de formule II
HNR¹R² (II)
dans laquelle R¹ et R² représentent, indépendamment l'un de l'autre, hydrogène ou un radical hydrocarboné le cas échéant substitué comprenant 1 à 100 atomes de carbone, est additionné d'eau avant l'irradiation par des micro-ondes, l'irradiation par des micro-ondes étant réalisée à des températures supérieures à 150°C.

4. Procédé selon l'une ou plusieurs des revendications 1 à 3, dans lequel l'irradiation par des micro-ondes a lieu à des pressions supérieures à la pression atmosphérique.

5. Procédé selon l'une ou plusieurs des revendications 1 à 4, dans lequel R³ représente un radical hydrocarboné comprenant 1 à 50 atomes de carbone, qui porte un ou plusieurs substituants choisis parmi les groupes C₁-C₅-alcoxy, poly(C₁-C₅-alcoxy), poly (C₁-C₅-alcoxy) alkyle, carboxyle, hydroxyle, ester, amide, cyano, nitrile, nitro, acide sulfonique et aryle comprenant 5 à 20 atomes de carbone, les groupes C₅-C₂₀-aryle pouvant porter des substituants choisis parmi les atomes d'halogène, les radicaux alkyle halogénés, les groupes C₁-C₂₀-alkyle, C₂-C₂₀-alcényle, C₁-C₅-alcoxy, ester, amide, hydroxy, hydroxyalkyle, cyano, nitrile et nitro.

6. Procédé selon l'une ou plusieurs des revendications 1 à 5, dans lequel R³ représente un radical hydrocarboné aliphatique, cycloaliphatique, aromatique ou araliphatique.

7. Procédé selon l'une ou plusieurs des revendications 1 à 6, dans lequel R³ comprend une ou plusieurs doubles liaisons.

8. Procédé selon l'une ou plusieurs des revendications 1 à 7, dans lequel R¹ et R² représentent, indépendamment l'un de l'autre, un radical hydrocarboné comprenant 1 à 100 atomes de carbone.

9. Procédé selon l'une ou plusieurs des revendications 1 à 8, dans lequel R¹ représente un radical hydrocarboné comprenant 1 à 100 atomes de carbone et R² représente hydrogène.

10. Procédé selon l'une ou plusieurs des revendications 1 à 9, dans lequel R¹ et R² ou les deux radicaux représentent, indépendamment l'un de l'autre, un radical aliphatique comprenant 1 à 24 atomes de carbone.

11. Procédé selon l'une ou plusieurs des revendications 1 à 10, dans lequel R¹ ou R² ou les deux radicaux portent des substituants choisis parmi les groupes hydroxy, C₁-C₅-alcoxy, cyano, nitrile, nitro et C₅-C₂₀-aryle.

12. Procédé selon l'une ou plusieurs des revendications 1 à 11, dans lequel R¹ ou R² ou les deux radicaux portent des groupes C₅-C₂₀-aryle et ceux-ci portent un ou plusieurs substituants choisis parmi les atomes d'halogène, les radicaux alkyle halogénés, les groupes C₁-C₂₀-alkyle, C₂-C₂₀-alcényle, C₁-C₅-alcoxy, ester, amide, cyano, nitrile et nitro.

13. Procédé selon l'une ou plusieurs des revendications 1 à 12, dans lequel R¹ et R² forment ensemble avec l'atome d'azote auquel ils sont liés un cycle.

14. Procédé selon l'une ou plusieurs des revendications 1 à 13, dans lequel R¹ et R² représentent, indépendamment l'un de l'autre, des radicaux de formule III
-(R⁴-O)ₙ-R⁵ (III)
où
R⁴ représente un groupe alkylène comprenant 2 à 6 atomes de carbone,
R⁵ représente hydrogène ou un radical hydrocarboné comprenant 1 à 24 atomes de carbone, et
n vaut un nombre entre 2 et 50.

15. Procédé selon l'une ou plusieurs des revendications 1 à 14, dans lequel R¹ et R² représentent, indépendamment l'un de l'autre, des radicaux de formule IV
-[R⁶-N (R⁷)]ₘ- (R⁷) (IV)
où
R⁶ représente un groupe alkylène comprenant 2 à 6 atomes de carbone ou des mélanges de ceux-ci, chaque R⁷ représente, indépendamment l'un de l'autre, hydrogène, un radical alkyle ou hydroxyalkyle comprenant jusqu'à 24 atomes de carbone, un radical polyoxyalkylène -(R⁴-O)ₚ-R⁵, ou un radical polyiminoalkylène -[R⁶-N(R⁷)]_{q}-(R⁷), R⁴, R⁵, R⁶ et R⁷ présentant les significations indiquées ci-dessus et q et p valant, indépendamment l'un de l'autre, 1 à 50, et
m vaut un nombre de 1 à 20, de préférence de 2 à 10, tel que par exemple trois, quatre, cinq ou six.

16. Procédé selon l'une ou plusieurs des revendications 1 à 15, dans lequel l'irradiation du sel par des micro-ondes a lieu dans un procédé par lots discontinu.

17. Procédé selon l'une ou plusieurs des revendications 1 à 16, dans lequel l'irradiation du sel par des micro-ondes a lieu dans un procédé continu.

18. Procédé selon la revendication 17, dans lequel l'irradiation du sel par des micro-ondes a lieu dans un tube de réaction dans une large mesure transparent aux micro-ondes.

19. Procédé selon la revendication 17 et 18, dans lequel l'irradiation du sel par des micro-ondes a lieu dans un tube de réaction, dont l'axe longitudinal se trouve dans le sens d'élargissement des micro-ondes d'un applicateur de micro-ondes monomodales.

20. Procédé selon l'une ou plusieurs des revendications 1 à 19, l'irradiation par des micro-ondes étant réalisée en présence de 0,5 à 200% en poids d'eau par rapport à la masse totale d'acide carboxylique et d'amine.

21. Procédé selon l'une ou plusieurs des revendications 1 à 20, dans lequel l'irradiation par des micro-ondes est réalisée à des températures supérieures à 180°C.
